# EUROPEAN PATENT SPECIFICATION

(11) **EP 1 633 887 B1**
(45) Date of publication and mention of the grant of the patent: **04.04.2007**
(21) Application number: 04739531.4
(22) Date of filing: 02.06.2004
(51) Int. Cl.: C12Q 1/68

(54) **FAST METHOD FOR DETECTING MICRO-ORGANISMS IN FOOD SAMPLES**
SCHNELL-VERFAHREN ZUR DETEKTION VON MIKROORGANISMEN IN LEBENSMITTELPROBEN
RAPIDE METHODE DE DETECTION DE MICRO-ORGANISMES DANS DES ECHANTILLONS ALIMENTAIRES

(30) Priority: 02.06.2003 EP 03447138
(43) Date of publication of application: 15.03.2006
(73) Proprietor: Check-Points Holding B.V., 6708 PW Wageningen (NL)
(72) Inventor: ANDREOLI, Peter, NL-4841 CM Prinsenbeek (NL); THIJSSEN, Joost, NL-6708 PW Wageningen (NL); ANTHONY, Richard, NL-1059 AG Amsterdam (NL); VOS, Pieter, NL-3911 VA Rhenen (NL); DE LEVITA, Wouter, NL-3572 CS Utrecht (NL)
(74) Representative: Kraft, Henricus Johannes
(86) International application number: PCT/EP2004/005951
(87) International publication number: WO 2004/106547

(56) References cited:
- WO-A-02/43937
- US-A1- 2002 182 598
- BLOHM D.H. AND GUISEPPI-ELIE A.: "New developments in microarray technology" CURRENT OPINION IN BIOTECHNOLOGY, vol. 12, no. 1, February 2001 (2001-02), pages 41-47, XP008040446 ISSN: 0958-1669
- GERRY N P ET AL: "Universal DNA microarray method for multiplex detection of low abundance point mutations" JOURNAL OF MOLECULAR BIOLOGY, LONDON, GB, vol. 292, no. 2, 17 September 1999 (1999-09-17), pages 251-262, XP004462280 ISSN: 0022-2836
- BUSTI ELENA ET AL: "Bacterial discrimination by means of a universal array approach mediated by LDR (ligase detection reaction)." BMC MICROBIOLOGY [ELECTRONIC RESOURCE]. ENGLAND 20 SEP 2002, [Online] vol. 2, no. 1, 20 September 2002 (2002-09-20), page 27, XP002262041 ISSN: 1471-2180 Retrieved from the Internet: URL:http:/www.biomedcentral.com/1471-2180/ 2/27> [retrieved on 2003-11-19]
- SMALL JACK ET AL: "Direct detection of 16S rRNA in soil extracts by using oligonucleotide microarrays" APPLIED AND ENVIRONMENTAL MICROBIOLOGY, WASHINGTON,DC, US, vol. 67, no. 10, October 2001 (2001-10), pages 4708-4716, XP002245295 ISSN: 0099-2240
- [Online] 19 November 2003 (2003-11-19), CEPARATION , HELMOND, THE NETHERLANDS , XP002262042 Retrieved from the Internet: URL:http:www.ceparation.com> [retrieved on 2003-11-19] cited in the application the whole document
- MIKHAILOVICH V ET AL: "Identification of rifampin-resistant mycobacterium tuberculosis strains by hybridization, PCR and ligase detection reaction on oligonucleotide microchips" JOURNAL OF CLINICAL MICROBIOLOGY, WASHINGTON, DC, US, vol. 39, no. 7, July 2001 (2001-07), pages 2531-2540, XP002970585 ISSN: 0095-1137

## Description

### Field of the invention

The present invention relates to a fast and efficient method for determining the presence of micro-organisms in a food sample.

### Background of the invention

In the manufacturing chain for food products, including dairy products, beverages and beer brewery, microbiological control and monitoring is of vital importance to validate the safety and quality of the beverages and the dairy products. The same considerations apply for water quality. Hence, the detection, identification, and characterization of micro-organisms are an important goal in analytical- and food microbiology as well as water control.

In many laboratories and research institutes new test methods to detect or screen micro-organisms are being developed (McCabe *et al*., 1999, Mol Genet Met **66**: 205-211; Cockerill and Thomas, 2002, ASM News **68** No 2: 77-83).

In these developments emphasis is being put on alternatives for the various incubation and pre-incubation step/methods in order to save time. Many new methods e.g. real time PCR using a Light-cycler (Roche Diagnostics Corp. Basel, Switzerland) will reduce the total screening time from five days to one day, but faster methods and especially more selective and discriminating methods to the type of micro-organism under investigation are needed.

In particular, the food industry is faced with the problem of detecting and identifying minute amounts of contaminating micro-organisms in large amounts of products being processed for consumption. It is a prerequisite that contaminating micro-organisms must be detected and identified fast in view of the large amounts of products and their associated costs. Obviously, contaminants must preferably be detected before the product has reached the end-user. Preferably, the product streams must be monitored continuously. It is therefore another goal to provide cheap detection tests. Microarrays are very efficient and reliable, but generally represent a large monitoring cost. Hence, versatile microarrays, which can be used for different tests and with a lower cost per microarray and/or test are needed.

The detection identification and characterization of microbes is an important goal in analytical microbiology. Culture-independent techniques represent a rapid and flexible means to study bacterial communities. The most comprehensive strategy to characterize microbial populations probably consists in 16S - 23S rDNA clones sequencing and phylogenetic reconstruction (Weissburg *et al.,* 1991, J.Bacteriol. **173**: 697-703; Anthony *et al.,* 2000, J.Clin.Microbiol: **38**: 781-788). The employment of group-specific nucleic acid probes complementary to 16S or 23S rRNA has provided a framework to study microbial populations in complex systems. Eukaryotic micro-organisms, such as yeast and fungi, may be identified in a similar way, e.g. by characterising the 18S and 28S rRNA.

RNA, however, is notoriously unstable. As a consequence, extreme quality measures have to be taken to preserve the characteristics of the RNA.

On the other hand, DNA is considered to be more stable. Moreover, whole genomic DNA-DNA hybridisation has been a cornerstone of microbial species determination. However, whole genomic DNA-DNA hybridisations is not widely used because it is not easily implemented.

Several hundreds of different species may qualify as a contaminating micro-organism. It is of prime importance to identify precisely the contaminating micro-organism.

The present invention aims at providing a specific method for accomplishing fast and specific identification of contaminating micro-organisms in large amounts of food stuffs. The invention further aims at the use of filters and microarrays in said method, as well as a kit for determining the presence of micro-organisms in a sample.

### Summary of the invention

The present invention relates to a specific method accomplishing fast and specific identification of contaminating micro-organisms in large amounts of food stuffs. A method has been developed based on the detection of species-specific and/or strain-specific nucleotide sequences that are uniquely identified and amplified and subsequently detected on a microarray using addressable Zipcode oligonucleotides and DNA microarray technology.

### Detailed Description of the Invention

The present invention relates to methods for collecting and identifying contaminating micro-organisms in food stuffs and in the control of water.

In particular, the present invention relates to a method for determining the presence of micro-organisms in a sample, comprising the steps of:
(a) capturing of said micro-organisms if present,
(b) extracting nucleic acids from said micro-organisms, said nucleic acids comprising target nucleic acids,
(c) performing a ligase detection reaction (LDR) on said target nucleic acids, comprising:
(c1) providing a pair of a first nucleic acid probe and a second nucleic acid probe, said first nucleic acid probe comprising a 3' located target-specific sequence I complementary to a distinct part of said target nucleic acid and said second nucleic acid probe comprising a 5' located target-specific sequence II complementary to a second part of said target nucleic acid located essentially adjacent to and 3' from said target-specific sequence I, wherein said first nucleic acid probe further comprises a 5' located primer binding section I (PBS(I)) and possibly a stuffer, and said second nucleic acid probe comprises a 3' located primer binding section II (PBS(II)) and possibly a stuffer, and optionally wherein the first nucleic acid probe and/or the second nucleic acid probe further comprises a region which is (i) essentially complementary to a corresponding region of a capture probe on a microarray and (ii) essentially non-complementary to said target nucleic acid (ZipComcode), and which is located in between the target specific sequence and the primer binding section;
(c2) incubating said target nucleic acid with said first nucleic acid probe and said second nucleic acid probe under conditions allowing hybridisation of complementary nucleic acids,
(c3) connecting any essentially adjacent probes, and
(c4) amplifying any connected probe nucleic acid, wherein amplification is initiated by binding of nucleic acid primer specific for a primer binding section, thereby providing amplified target nucleic acids,
(d) hybridising the amplified target nucleic acids of step (c) to a capture probe, which is present on a flow-through microarray, and optionally comprises a region essentially complementary to the ZipComcode (Zipcode), and,
(e) detecting the hybridised target nucleic acids of step (d), whereby the presence of a micro-organisms is determined.

In the present specification and the appended claims, the singular forms "a", "an", and "the" include the plural references, and vice versa, unless the context clearly indicates otherwise. Unless defined otherwise, all technical and scientific terms used herein have the same meaning as commonly understood by one of ordinary skill in the art.

In general, a sample or specimen will be taken as a part of anything, e.g. food stuffs, dairy products, beverages and beer being produced presented for inspection, or shown as evidence of the quality of the whole.

The present method is applicable to the micro-organisms which are known to contaminate food stuffs, dairy products, beer and other beverages, for example the micro-organisms presented in Table 1. As such, the present invention relates to a method for determining the presence of micro-organisms in a sample, comprising the steps of collecting said micro-organisms if present, extracting nucleic acids from said micro-organisms, specifically amplifying said nucleic acids, and analysing the amplified, nucleic acids, whereby the presence of said micro-organisms is determined. In addition, the present invention relates to a method for determining the presence of micro-organisms in a sample, comprising the steps of collecting said micro-organisms if present, extracting nucleic acids from said micro-organisms, and analysing the nucleic acids, whereby the presence of said micro-organisms is determined.

As will be evident, the present invention relates to a method as described herein, wherein said micro-organism is selected from the group consisting of eukaryotic and/or prokaryotic micro-organisms as well as viruses. The micro-organism may be selected from the group comprising algae, archaea, bacteria, viruses, nematodes, protozoa, microsporidae and fungi including yeasts, moulds and mycorrhizae.

Similarly, it will be appreciated that the present invention relates to a method as described herein, wherein said micro-organism is selected from the group consisting of food borne and waterborne micro-organisms.

In this respect, the present invention relates to a method as described herein, wherein said micro-organism is selected from the bacteria group consisting of *Escherichia, Salmonella, Shigella, Mycobacterium, Lactobacillus, Lactococcus, Listeria, Leuconostoc, Bacillus, Staphylococcus, Clostridium, Vibrio, Enterococcus, Enterobacter, Yersinia, Legionella, Campylobacter, Streptococcus, Micrococcus, Pseudomonas, Flavobacterium, Alcaligenes, Microbacterium, Acinetobacter,* and *Enterobacteriaceae*/*Coliforms* and from the moulds *Aspergillus, Neurospora, Geotrichum, Blakeslea, Penicillium, Rhizomucor, Rhizopus* and *Trichoderma,* and from the yeasts *Kluyveromyces, Candida, Hansenula, Rhodotorula, Torulopsis, Trichosporon* and *Saccharomyces.* Moreover, the present invention relates to a method as described herein, wherein said micro-organism is selected from the group consisting of a (sub)species from the genus *Escherichia*, *Salmonella, Shigella, Mycobacterium, Lactobacillus, Lactococcus, Listeria, Leuconostoc, Bacillus, Staphylococcus, Clostridium, Vibrio, Enterococcus, Enterobacter, Yersinia, Legionella, Campylobacter, Micrococcus, Pseudomonas, Flavobacterium, Alcaligenes, Microbacterium, Acinetobacter, Enterobacteriaceae*/*Coliforms,* and *Streptococcus,* and from the moulds *Aspergillus, Neurospora, Geotrichum, Blakeslea, Penicillium, Rhizomucor, Rhizopus* and *Trichoderma,* and from the yeasts *Kluyveromyces, Candida, Hansenula, Rhodotorula, Torulopsis, Trichosporon* and *Saccharomyces,* e.g. as set out in Table 1.

In order to increase the amount of micro-organisms present in a sample, said micro-organisms, if present, may be grown on media. Accordingly, the present invention relates to a method as described herein, wherein said method, for instance step (a) of above, is preceded by an enrichment of micro-organisms, comprising (i) growth of said micro-organisms on selective media, or (ii) growth of said micro-organisms on non-selective media. Growth of said micro-organisms on selective media will preferably favour the growth of micro-organisms of interest, while the growth on non-selective media will sustain growth of most micro-organisms, e.g. not especially favouring the growth of a particular micro-organism.

Standard laboratory techniques for collecting micro-organisms in large amounts of solutions or solid materials are time consuming. In order to circumvent these time-consuming pre-enriching step according to standard laboratory techniques, such as advocated by AOAC (Association of Analytical Communities), the present invention immediately collects the contaminating micro-organism by concentrating it. Accordingly, the present invention relates to a method as described herein, wherein said method, for instance step (a) of above, is preceded by an enrichment of micro-organisms, comprising concentrating the micro-organisms. The said collecting and capturing may be performed by means of centrifugation, filtration, such as filtering of an aqueous or liquid solution, whereby all particles larger than the sieving size are being captured, sedimentation, electrostatic forces, coagulation, flocculation, capturing of micro-organisms by antibodies, and/or capturing of micro-organisms by ligands.

In addition, the method according to the present invention may apply microfiltration for collecting or capturing the contaminating micro-organisms, e.g. Micro Analytical Screen (MAS) method. The ultimate goal in membrane microfiltration is to achieve a low flow resistance, a high chemical resistance and a well controlled pore size distribution of the membrane filters, in order to obtain a high operational flux, long standing times (e.g. a long life/operation time of the microsieve) and good separation behaviour. Preferably, the microsieve filters according to the present invention are characterised by thin membrane layers with uniformly sized pores. For most applications, the membrane layer is sustained by a support. A microsieve having a relatively thin filtration or sieving layer with a high pore density and a narrow pore size distribution on a macroporous support will show a satisfactory to good or even excellent separation behaviour and a high flow rate. In very dilute suspensions, it will be important to have a fast determination of the kind and concentration of particles, such as for example fruit juices contaminated with micro-organisms. The low flow resistance of the microsieve allows a large amount of liquid to pass through the filter in a small amount of time, whereby the contaminating micro-organisms (if present) are concentrated on a very small surface (20 -100 mm²). This fast concentration of the contaminating micro-organisms adds in simplifying and the quality of the subsequent analysis of these micro-organisms.

In this regard, the present invention relates to cross-flow microfiltration as described by Daufin *et al.* (2001), (Daufin *et al*. (2001) Trans IchemE, 79: 89-102). Cross-flow microfiltration may be used for the removal of microparticles, such as contaminating micro-organisms from many different fluids. Accordingly, cross-flow microfiltration can be industrially applied in food, water and bioprocesses.

Also, the present invention applies a new microfiltration technology, which has been described by the patent application WO 02/43937 (by Aquamarijn Holding Ltd.). In addition, the present invention applies a new microfiltration technology, which has been developed by CEPAration B.V. (Helmond, The Netherlands). CEPAration produces and develops hollow fibre ceramic membranes and modules for applications ranging from microfiltration to high temperature gas separation. These products combine the advantages of polymeric hollow fibre membranes with the outstanding and durable properties of ceramic membranes. CEPAration has its Production & Development site in Helmond, The Netherlands (http://www.ceparation.com).

Accordingly, the present invention relates to a method as described herein, wherein said filtration is performed by using an Aquamarijn® filter or a CEPAration® filter. For instance, silicon nitride may be used as membrane, and silicium as carrier, or the filter may comprise a hollow fibre ceramic membrane. The size of pores may for instance be between 0.5 and 1.2 micron or between 0.15 and 1.4 micron.

It will be understood that the present invention relates to a method as described herein, wherein said concentrating is followed by separating the micro-organisms from the remainder of the sample. In addition, concentrating and separating may be performed simultaneously.

A wide variety of colouring and/or staining techniques can be used in order to improve the recognition of the micro-organisms on the microsieve surface. Microsieves are preferably inert which makes it possible to use all present staining agents and chemicals without colouring or attacking the microsieve surface. Said microsieve may be used again.

The presented Micro Analytical Screen (MAS) method may also be applied for the quality control of water in general and drinking water in particular on the presence of contaminating micro-organisms, such as for example, *Cryptosporidium*, *Escherichia coli* and *Legionella*. Also in the meat industry, the MAS method can be applied to trace contaminating micro-organisms, such as for example, *Campylobacter* and *Salmonella* contaminations.

In order to characterise the contaminating micro-organism, the present invention may employ known techniques identifying the nucleic acid of the micro-organism at issue. The present invention relates preferably to the multiplexed amplification and labelling technique described below.

The term "nucleic acid" as used herein means a polymer composed of nucleotides, e.g. deoxyribonucleotides or ribonucleotides. The terms "ribonucleic acid" and "RNA" as used herein means a polymer composed of ribonucleotides. The terms "deoxyribonucleic acid" and "DNA" as used herein means a polymer composed of deoxyribonucleotides. The terms "oligonucleotide", "primer" and "probe" as used herein denotes single stranded nucleotide multimers of from about 10 to about 100 nucleotides in length. The term "polynucleotide" as used herein refers to single or double stranded polymer composed of nucleotide monomers of from about 10 to about 100 nucleotides in length, usually of greater than about 100 nucleotides in length up to about 1000 nucleotides in length.

It will be understood that the present invention relates to a method as described herein, wherein said nucleic acids are chosen from the group consisting of DNA, rRNA, tRNA, mRNA, total RNA and tmRNA (dual tRNA-like and mRNA-like nature; also known as 10Sa RNA or SsrA).

In order to characterise the nucleic acid from a contaminating micro-organism, i.e. the target nucleic acid, said nucleic acid is normally isolated from the contaminating micro-organism after said organism has been collected or captured. In general, isolation of nucleic acids from micro-organisms requires as one of the first steps the lysis of said micro-organism. It will be apparent that the cell lysis strategies employed are dependent of the nature of the contaminating micro-organisms. In general, a treatment with a lysozyme, a pectinolytic, or a mechanical treatment such as sonication or a bead beater can be used for lysing the cells. A customary procedure is the direct injection of bacterial samples into a hot phenol solution, such as described by Selinger *et al.* (2000, Nature Biotechnol. **18**, 1262-1268). Alternatively, cells can be quickly frozen in liquid nitrogen and mechanically broken before isolation with an acid phenol solution. Classical methods for isolating nucleic acids relating to combinations of enzymatic degradation, organic extraction and alcohol and/or salt precipitation are well known in the art, and contemplated by the present invention. In this regard, the techniques for isolating ribonucleic acids are described in Current Protocols in Molecular Biology, Wiley & Co, USA. The present invention also relates to rapid small scale purification of DNA and RNA from clinical samples. The latter method may be based on the lysing and nuclease inactivating properties of the chaotropic agent guanidinum thiocyanate (GuSCN) and the nucleic acid-binding properties of silica particles or diatoms in the presence of this agent, such as described by Boom *et al.* (1999; J. Clin. Microbiol. 37: 615-619).

Most microbial mRNA species only have a half-life of minutes, mainly due to the activity of RNases. Therefore, the speed required to stabilize the RNA population, i.e. to arrest or decrease RNA degradation, becomes crucial. In this respect, various inhibitors of ribonuclease activity, such as, for example, diethylpyrocarbonate, aurintricarboxylic acid, etc, may be employed in RNA isolation procedures, and belong to the common, general knowledge regarding isolation of RNA, and are incorporated herein. The lysis of said contaminating micro-organism may be performed before or after stabilising the nucleic acid population. The present invention relates also to a stop solution containing ethanol and phenol, as has been described for the isolation of total RNA from *E*.*coli* (Ye *et al*., 2001, J.Microbiol. Methods 47, 257-272). This stop solution may be used successfully for other Gram negative bacteria. In addition, the present invention contemplates the use of the RNAlater® solution (Ambion and Qiagen). The main advantage of the latter solution is its rapid stabilisation of the mRNA population, allowing the samples to be stored for a long period of time under appropriate conditions prior to RNA isolation. It is especially useful for the collection of samples when immediate isolation of RNA is not possible.

Accordingly, the present invention relates to a method as described herein, wherein said step of extracting nucleic acids from said micro-organisms comprises lysing the micro-organisms.

Also, the present invention relates to a method as described herein, further comprising inactivating RNAses.

As most of the mRNAs of bacteria do not have a poly A+ tail and are therefore difficult to separate from the total RNA, an enrichment step may be used. The present invention relates to an enrichment step for mRNA; by removing the ribosomal RNA as described by Affymetrix (http: // www.affymetrix.com/index.affx). In addition, the present invention incorporates a method to isolate *E.coli* mRNA by polyadenylating it in crude cell extracts with poly A+ polymerase I from *E.coli* and purifying it by oligo-dT chromatography as described by Wendisch *et al.* (Wendisch *et al.* (2001) Anal. Biochem. 290: 205 - 213).

A variety of RNA isolation kits are available from different commercial sources, e.g. from Ambion, Qiagen, Sigma-Aldrich and others, which may successfully be used in the method of the present invention.

As described above for isolating RNA, in isolating DNA the method to lyse the micro-organism depends on the type of micro-organism, e.g. moulds, fungi, yeast, Gram negative or Gram positive bacteria. In this regard, the techniques for isolating DNA are described in Current Protocols in Molecular Biology, Wiley & Co, USA. A convenient method is to immerse the cells in boiling water. For example, in the case of Gram positive organisms, such as *Lactobacillus,* the cells may suspended in a buffer, such as STE buffer (100mM NaCl, 50mM Tris-HCl, 10mM sodium EDTA, pH 7.5) and incubated at 37°C with lysozyme (e.g. 10 mg/ml) for an appropriate amount of time, e.g. 15 minutes. In the case of Gram negative organisms, such as for example, *Salmonella,* genomic DNA from the samples may be extracted and purified using Genomic tips (Qiagen) using a Genomic DNA buffer set (Qiagen). In case of moulds or fungi, such as for example *Aspergillus,* the cells may be suspended in OM-buffer and treated with a pectinolytic, e.g. Glucanex® (Novozymes, Denmark). Accordingly, the present invention relates to a method as described herein, wherein said lysing is chosen from the group consisting of a treatment with a lysozyme, a pectinolytic, or guanidinium thiocyanate or by a mechanical treatment such as sonication or the use of a bead beater, by injecting the micro-organisms in hot phenol, and snap freezing the micro-organisms in liquid nitrogen followed by a mechanical treatment.

A variety of genomic DNA isolation kits are available from different commercial sources, e.g. from Gentra, Promega, Qiagen and others, which may successfully be used in the methods of the present invention.

The concentration of the isolated nucleic acid may be estimated by spectrophotometry at 260 nm.

After nucleic acids have been isolated from the contaminating micro-organisms, said nucleic acids need to be analysed. In general, only minute amounts of contaminating micro-organisms are present. Therefore, the isolated nucleic acids or a specific portion thereof, i.e. the target nucleic acid, may be amplified. In case of the target nucleic acid being RNA, said RNA may first be converted to cDNA before analysis. It will be understood that the terms "amplified nucleic acids" and "amplified nucleic acid mixture" as used throughout the invention have essentially the same meaning.

Therefore, the present invention relates to a method as described herein, wherein said nucleic acid is rRNA, tRNA, mRNA, total RNA, or tmRNA and wherein said rRNA, tRNA, mRNA, total RNA, or tmRNA is converted to cDNA, e.g. by the activity of a reverse transcriptase, as is well known in the art.

Various techniques are known by the person skilled in the art to amplify DNA and/or cDNA. All of these techniques are contemplated by the present invention. Accordingly, the present invention relates to a method as described herein, wherein said nucleic acid is DNA and/or cDNA, and wherein said DNA and/or cDNA is amplified using an amplification technique such as bDNA, Hybrid capture, SDA, TMA, PCR, LCR, TAS, 3SR, NASBA and Qβ amplification, as explained in Versalovic and Lupski 2002, Trends Microbiology 10: S15-S21.

The present invention especially contemplates multiplex amplification, such as multiplex PCR. Multiplex amplification, such as multiplex PCR, allows amplification, and thus analysis of two or more targets simultaneously. This amplification technique is used for genetic screening, micro satellite analysis, and other applications where it is necessary to amplify several products in a single reaction. By routine experimentation the person skilled in the art will be able to optimize the reaction conditions, in view of having multiple primer pairs in a single reaction, which may increase the likelihood of primer-dimers and other nonspecific products that may interfere with the amplification of specific products. In addition, the concentrations of individual primer pairs often need to be optimized since different multiplex amplicons are often amplified with differing efficiencies, and multiple primer pairs can compete with each other in the reaction. The person skilled in the art will make similar considerations and optimize the conditions for the other amplification techniques described above for multiplex amplifications, i.e. amplification of more than one target.

In addition, the present invention relates to the direct amplification of RNA, such as for example via a modified Tyras method, wherein a primer/probe comprising a RNA polymerase recognition site and recognition site complementary to the target nucleic acid is used.

After isolating the target nucleic acid, probes and/or primers are hybridised to the said target nucleic acid. The primers may be used to amplify the said target nucleic acid. Alternatively, the probes may be ligated and may be amplified with primers specifically recognising regions on said probes (see below). The probes and/or primers may be labelled. Also, the label may be incorporated during the amplification step or attached after amplification. Accordingly, the present invention relates to a method as described herein, wherein the amplified nucleic acid is labelled. Virtually any label that produces a detectable, quantifiable signal and that is capable of being attached to the amplified nucleic acid, can be used in conjunction with the methods and arrays of the invention (see infra). Suitable labels include, by way of example and not limitation, radioisotopes, fluorophores, chromophores, chemiluminescent moieties, etc. In embodiments where the label is attached to the amplified nucleic acid, the label can be attached to any part of the nucleic acid, including the free terminus or one or more of the bases. Preferably, the position of the label will not interfere with hybridisation, detection or other post-hybridisation modifications of the labelled nucleic acid. A variety of different protocols may be used to generate the labelled nucleic acids, as is known in the art, where such methods typically rely on the enzymatic generation of labelled nucleic acid using an initial primer and template nucleic acid. Labelled primers can be employed to generate the labelled amplified nucleic acid. Alternatively, label can be incorporated into the nucleic acid during first strand synthesis or subsequent synthesis, labelling or amplification steps in order to produce labelled amplified nucleic acid. Label can also be incorporated directly to mRNA using chemical modification of RNA with reactive label derivatives or enzymatic modification using labelled substrates. Representative methods of producing labelled amplified nucleic acid are disclosed in U. S. Application Serial nos.: 08/859,998; 08/974,298; 09/225,998.

The amplified nucleic acids may be labelled, for example, by the labels and techniques described supra. Alternatively, they may be labelled by any other technique known in the art. Preferred techniques include direct chemical labelling methods and enzymatic labelling methods, such as kinasing and nick-translation. Accordingly, the present invention relates to method as described herein, wherein the amplified target nucleic acid may be labelled during amplification, or the amplified target nucleic acid may be labelled after amplification.

A variety of different labels may be employed, where such labels include fluorescent labels, phosphorescent labels, isotopic labels, enzymatic labels, particulate labels, etc. For example, suitable labels include fluorochromes, e.g. fluorescein isothiocyanate (FITC), rhodamine, such as rhodamine 123, R6G, IRDyes^{™}, Texas Red, phycoerythrin, allophycocyanin, 6-carboxyfluorescein (6-FAM), 2',7'-dimethoxy-4',5'-dichloro-6-carboxyfluorescein (JOE), 6-carboxy-X-rhodamine (ROX), TET, JOE, NED, (ET-)ROX, 6-carboxy-2',4',7',4,7-hexachloro-fluorescein (HEX), 5-carboxyfluorescein (5-FAM) or N,N,N',N'-tetramethyl-6-carboxy-rhodamine (TAMRA), fluor 488^{™}, cyanine dyes, e.g. Cy5, Cy3, Cy2, BODIPY dyes, e.g. Biodipy^{™} 630/650, Biodipy 530, Biodipy^{™} FL, Alexa such as Alexa542, Alexafluor^{™} 532, etc. Suitable isotopic labels include radioactive labels, e.g. ³²P, ³³P, ³⁵S, ³H. Other suitable labels include size particles that possess light scattering, fluorescent properties or contain entrapped multiple fluorophores. The label may be a two stage system, where the primer and/or probe is conjugated to biotin, haptens, etc. having a high affinity binding partner, e.g. avidin, specific antibodies, etc. The binding partner is conjugated to a detectable label, e.g. an enzymatic label capable of converting a substrate to a chromogenic product, a fluorescent label, an isotopic label, etc.

As such, in certain embodiments, the primers directed to different target nucleic acids may be differentially labelled. By "differentially labelled" and "contain a different label" is meant that the primers directed to different target nucleic acids are labelled differently from each other such that they can be simultaneously distinguished from each other.

An embodiment of the invention relates to the combination of (1) multiplex Ligase Detection Reaction (LDR) and (2) multiplex Polymerase Chain Reaction (PCR). The Ligase Detection Reaction (LDR) is a sensitive assay for detecting Single Nucleotide Polymorphisms (SNPs), as described by Favis *et al.,* (2000, Nature Biotechnology 18: 561 - 564). A difference in a single nucleotide along the 16S rRNA may be employed to distinguish between sequences of different micro-organisms, as described by Busti *et al.* (2002, BMC Microbiology 2: 27-39). Similarly, single nucleotide differences along the 18S, 23S or 28S rRNA may be employed to distinguish between sequences of different micro-organisms. A set of two probes (probe I and II) may be designed, based on the target sequence to be detected, of which at least a part is known. Both probes contain a region at the end (the 3' and the 5' end of the respective probes I and II) that is capable of hybridizing to the known section of the target sequence. In other words, one probe (probe I) comprises a region Ir specifically hybridising to a target region, said region Ir being located at the ultimate 3' end of probe I. Said probe I further comprising a primer binding section (PBS(I)), located 5' from the region Ir. Said probe I and/or II may contain a stuffer region. For instance, said stuffer region on probe I may be located between region Ir and PBS(I). The probe II comprises a region IIr specifically hybridising to a target region, said region IIr being located at the ultimate 5' end of probe II. Said probe II further comprising a primer binding section (PBS(II)), located 3' from the region IIr. Probe I or Probe II may further comprise a ZipComcode, located in-between the region Ir and PBS(I) or the region IIr and PBS(II), respectively. The ZipComcode (ZCc) is a unique sequence for identification of the eventually amplified products. The ZCc will hybridise to its complement the Zipcode, present on for instance a microchip (capture probe; see below). Upon hybridisation, the target region Ir of probe I is located adjacent to the target region IIr of probe II. The ZCc and the PBSs are located at the ends of the probes, and are not capable of hybridising to the target sequence. When both probes are hybridized to the target sequence, and are located adjacent to each other, the probes can be ligated using a ligase, such as for example *Pfu* DNA ligase. After ligation, the ligated probes may be amplified using at least one primer that is capable of hybridizing to a primer binding section. Preferably, amplification is carried out by PCR, using probe I with a PBS(I) which differ from probe II with PBS(II). Hence, primer I binding to the region characterised by PBS(I) will differ from primer II binding to the region characterised by PBS(II). It will be appreciated that if primer I comprises a sequence substantially complementary to PBS(I), then primer II comprises a sequence substantially identical to PBS(II), and vice versa, that if primer I comprises a sequence substantially identical to PBS(I), then primer II comprises a sequence substantially complementary to PBS(II). One of the primers may be labelled, for example at its 5' end. Preferably, the first primer is labelled at its 5' end. Also, the second primer may comprise a ZipComcode located at the 5' end. As such, in a multiplex, the method may operate using one common primer, e.g. hybridising to PBS(I), and one probe specific primer, e.g. hybridising to PBS(II). It will be appreciated that the common primer may hybridise to PBS(II), while the probe specific primer hybridises to PBS(I). In a further embodiment, probe I contains a label.

Hence, in the method according to the present invention said nucleic acid and/or cDNA may be amplified using the Ligase Detection Reaction, comprising a first nucleic acid probe complementary to a distinct part of said target nucleic acid and a second nucleic acid probe complementary to a second part of said target nucleic acid located essentially adjacent to said distinct part of said target nucleic acid, wherein said first nucleic acid probe further comprises a 5' located primer binding section and possibly a stuffer, and said first or said second nucleic acid probe comprises a 3' located ZipComcode tag which is essentially non-complementary to said target nucleic acid and a primer binding section, which in case of said second nucleic acid probe is located 3' from the ZipComcode. The method further comprising incubating said nucleic acid and/or cDNA allowing hybridisation of complementary nucleic acids, connecting any essentially adjacent probes (by ligating), and amplifying any connected probe nucleic acid, wherein amplification is initiated by binding of nucleic acid primers specific for primer binding sections.

Thus, the present invention relates to a method as described herein, wherein said connecting step comprises the use or activity of a ligase, such as T4 ligase, or a thermostable ligase such as *Taq* DNA ligase, *Pfu* DNA ligase, Tth DNA ligase or Ampligase^{™}.

A typical structure of ligated probes is the following:
I: 5'-PBS(I) - [stuffer] - target specific region I --- target specific region II - [stuffer] - [ZCc] - [stuffer] - PBS(II)-3', or
II: 5'-PBS(I) - [stuffer] - [ZCc] - [stuffer] - target specific region I --- target specific region II - [stuffer] - PBS(II)-3'.

The typical structure of the ligated probe after amplification are:
I: 5'-[Label] - PBS(I) - [stuffer] - target specific region I - target specific region II - [stuffer] - [ZCc] - [stuffer] - PBS(II)-3', and
II: 5'- PBS(I) - [stuffer] - [ZCc] - [stuffer] - target specific region I - target specific region II - [stuffer] - PBS(II) - [Label] -3'.
(the regions between square brackets are optional)

Accordingly, the present invention relates to a method as described herein, wherein said probe I and/or probe II comprises a stuffer region. In this regard, a stuffer region is intended to part structural regions, such as the PBS, the ZCc, the Ir or IIr, thereby avoiding or minimizing steric hindrance.

As already set out above, it will be appreciated that the label may be attached to at least one of the primers and/or probes, or in the alternative, may be incorporated during amplification. The label is for instance a fluorescent label. Accordingly, the present invention relates to a method as described herein, wherein at least one primer contains a label, and preferably a fluorescent label.

It will be appreciated that RNA-DNA hybrids can act as substrates for T4 DNA ligase, as described by Charani Ranasinghe and Andrew A. Hobbs Affiliations in Elsevier Trends Journals Technical Tips Online, [Tip]01519 "A simple method to obtain the 5' ends of mRNA sequences by direct ligation of cDNA-RNA hybrids to a plasmid vector".

In the alternative, one of the probes I or II or primers contains an RNA polymerase binding site. The ligated probes are subsequently amplified by the activity of an RNA polymerase, e.g. T4-, T7- or SP6 RNA polymerase.

Genetic markers represent (mark the location of) specific loci in the genome of a species or closely related species. A sampling of different genotypes at these marker loci reveals genetic variation. The genetic variation at marker loci can then be described and applied to diagnostics and the like. Genetic variation between species may be ascribed to single nucleotide substitutions in the DNA or the 16S, 18S, 23S and/or 28S rRNA. The target binding region of the probes may be adapted correspondingly. For example, a set of four probes I may be provided, each of which comprising a different 3' ultimate nucleotide, e.g. probe I-A, probe I-C, probe I-G and probe I-T, containing the nucleotide A, C, G and T respectively at its 3' end. It will then be advantageous if the PBS of each probe I, is specific and corresponds to said ultimate nucleotide. In other words, the PBS of each probe I hybridises to a different primer I. Hence, the present invention contemplates probe I-A with PBS(I-A), which hybridises to the corresponding primer I-A, probe I-C with PBS(I-C), which hybridises to the corresponding primer I-C, probe I-G with PBS(I-G), which hybridises to the corresponding primer I-G, and probe I-T with PBS(I-T), which hybridises to the corresponding primer I-T. Each of said primers I-A, I-C, I-G and I-T may comprise a different label. It will be appreciated by the person skilled in the art that variations on this theme are conceivable, e.g. where the genetic marker is located within the target region of the probes, or on the ultimate 5' end of probe II. In the case that the genetic marker is located in probe II, the PBS(II) may be adapted as described above for probe I. Furthermore, the PBS, i.e. PBS(I) and PBS(II) may be identical or different.

Accordingly, the present invention relates to a method as described herein, wherein probe I, i.e. said first nucleic acid probe, and/or probe II, i.e. said second nucleic acid probe, specifically hybridises to a genetic marker.

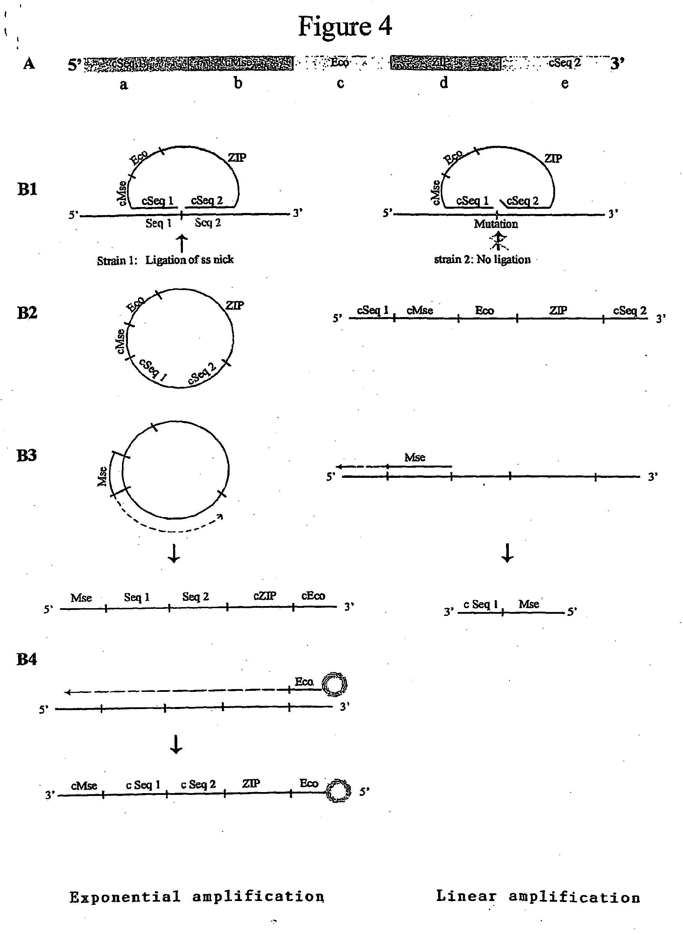

Accordingly, the present invention relates to a method as described herein, wherein 4 variants of probe I, i.e. said first nucleic acid probe, are provided, said 4 variants being substantially identical, except that each of the 4 variants containing a different nucleotide at its ultimate 3' end. Also, the present invention relates to a method as described herein, wherein each of said 4 variants containing a different primer binding section I. In a further embodiment, the present invention relates to a method as described herein, wherein at least two groups of pairs of first and second nucleic acid probes are provided, wherein each group of first and second nucleic acid probes hybridises to a specific target nucleic acid, and comprises a specific primer binding site I and/or II. In a further aspect, the invention relates to a method as described herein, wherein at least two groups of pairs of first and second nucleic acid probes are provided, wherein each group of first and second nucleic acid probes hybridises to a specific target nucleic acid, and the first nucleic acid probe of each group comprises a specific ZipComcode. As such, the ZipComcode may be located on the first nucleic acid probe in between the target-specific sequence I and the primer binding sequence I. In a further aspect, the first nucleic acid probe is attached or coupled with its 5' end to the 3' end of said second nucleic acid probe, possibly via a stuffer region (see for instance Figure 4, which provides a generalized concept). It will be understood that a circular probe results after ligating target-specific sequence I to target-specific sequence II.

Accordingly, the present invention relates to a method as described herein, wherein each of the primers binding to each of the different primer binding section I of said 4 variants contains a different fluorescent label.

Accordingly, the present invention relates to a method as described herein, wherein a set of two adjacent probes is provided for the micro-organisms as defined supra. Also, these probes may be coupled.

The method described herein relates to the simultaneous detection of various contaminating micro-organisms, by providing at least one set, and preferably more than one set of two probes, specifically designed to identify and/or characterise the presence of a contaminating micro-organism (multiplex). The different sets of probes should preferably not cross-hybridise, while on the other hand the melting temperature Tm of the different sets of probe/primers is about similar, e.g. varying not more than about 12°C. Commonly available computer programmes, such as Probe Match, Michigan State University, East Lansing, Michigan USA, Oligo 5.0 software (PE Biosystems, Foster City, California, USA), and using Clustal W Algorithm, may facilitate the design of specific probes. Preferably, the primers/probes have a melting temperature Tm between about 37-85 °C, or 50-80 °C, or 55-75 °C, or 60-72 °C. As such, the present invention relates also to multiplex amplification (see above).

In another aspect, the present invention relates to a method as described herein, comprising providing at least one set of two primers, wherein the first primer (primer A) comprises a 5' located label and a region A specifically hybridising to a first target nucleic acid region, said region A being located at the ultimate 3' end of primer A, and wherein the second primer (primer B) comprises a 3' located ZipComcode and a region B specifically hybridising to a second target nucleic acid region, said region B being located at the ultimate 5' end of primer B; the first target nucleic acid region target region being located 3' adjacent to the second target nucleic acid region; incubating said target nucleic acid with said primer A and said primer B under conditions allowing hybridisation of complementary nucleic acids; connecting any essentially adjacent primers; and hybridising the connected primers to a capture probe, which comprises a region essentially complementary to the ZipComcode, and which is present on a flow-through microarray. As such, said primer A may specifically hybridise to a genetic marker. In a further aspect, 4 variants of primer A are provided, said 4 variants being substantially identical, except that each of the 4 variants contain a different nucleotide at its ultimate 3' end, and each of the 4 variants contain a different fluorescent label.

After collecting the contaminating micro-organism, isolation of its nucleic acid and amplification, the amplified nucleic acids or amplified nucleic acid mixture may be analysed. A convenient method to analyse said amplified nucleic acid or said amplified nucleic acid mixture is by determining the sequence thereof. Techniques to determine the sequence of nucleic acids are well known in the art. Accordingly, the present invention relates to a method as described herein, wherein the analysis comprises determining the sequence of the amplified nucleic acid mixture. Said sequence may be determined via enzymatic, chemical or physical means. The sequence determined of the contaminating organism may be compared with sequences stored in a databank. Also the step of analysing in the method for characterising micro-organisms possibly present in a sample according to the present invention, may comprise providing a computer readable medium carrying computer output data having a database characterising micro-organisms based on nucleotide sequences, providing a computer and algorithm, processing the computer output data to determine the micro-organism.

Another convenient method to characterise the contaminating micro-organisms is by performing an amplified fragment-length polymorphism analysis (AFLP) as described by, for instance, Vos *et al*. (1995 Nucleic Acids Research 23:4407-4414). In an embodiment, AFLP is used to identify differentially amplified nucleic acids, which are then converted into polynucleotide probes that map to polymorphisms. The differentially amplified AFLP DNAs are converted into polynucleotide probes by isolating individual polymorphic AFLP fragments from a mixture of fragments in an AFLP amplification product, followed by using the isolated fragments as polynucleotide probes in hybridisations with immobilised DNA amplification products. Further representative methods of identifying AFLPs are disclosed in International Application Serial No: WO 98/30721.

Also, the detection and identification of micro-organisms by high-throughput screening requires easy to use, species specific markers. Accordingly, the present invention contemplates AFLP analysis via the generation of genomic fragments (polymorphic AFLP fragments) by digesting genomic DNA with one or more restriction enzymes. These genomic fragments may be differentiated by size. Polymorphic AFLP fragments of interest may be selected and analysed, for instance by cloning and sequence determination. The resulting fragments may be blasted against several databases, for instance the IECB University of Vienna: (www.probebase.net), the TIGR (www.tigr.org/tdb/mdb/mdbcomplete.html) or the Genbank (www.ncbi.nlm.nih.gov) databases. All obtained sequences showing internal or external homologies and falling outside the desired Tm range, for instance 50 - 80 °C, are eliminated. The remaining sequences which are species-specific are named '"signature sequences/tags" These signature sequences/tags can be applied both as primers as well as capture probes in the amplification step and in the microarray identification.

Accordingly, the present invention relates to a method as described herein, wherein said nucleic acid is DNA, and wherein said DNA is subjected to AFLP.

It will be appreciated by the person skilled in the art that the AFLP^{(™)} markers for genetic map construction in plants and micro-organisms may be used in the present invention, and may accelerate genome analysis. Preferably, the detection and identification of food and/or water borne micro-organisms by high-throughout screening may be done by easy to use, species-specific markers.

Another embodiment of the present invention relates to the use of arrays, e.g. microarrays, for the analysis of the amplified nucleic acids. Arrays may contain thousands of DNA spots. A single array has the potential for a broad identification capacity, i.e. many different contaminating micro-organisms may be analysed on one microarray, in one go.

In addition, the method of the invention does not require laborious cross-hybridisations and may provide an open database of hybridisation profiles, avoiding the limitations of traditional DNA-DNA hybridisations.

In the presence of a perfectly matching template, the probes may be ligated by the action of a DNA ligase. After ligation, said probes may be amplified. Next, the ligated probes, which may be or may be not amplified, are brought into contact with a capture probe, under hybridising conditions. Hybridising conditions are well known in the art, or may be determined without difficulty by the person skilled in the art, see e.g. "Molecular Cloning: A Laboratory Manual" Second Edition (Sambrook et al.,1989) and "Current Protocols in Molecular Biology" (F. M. Ausubel et al., eds., 1987, and periodic updates). Said capture probe comprises a complementary sequence relative to the target nucleic acid sequence, or a part thereof, such as the ZCc. The capture probes may be located on a microarray. Hence, the microarray comprises the complementary sequences of the target nucleic acid sequences, i.e. the capture probe. The location of the capture probe on the microarray is known.

Accordingly, the present invention relates to a method as described herein, wherein said analysing comprises hybridising the amplified nucleic acids or said amplified nucleic acid mixture to a capture probe, said capture probe hybridising specifically to said amplified nucleic acids or said amplified nucleic acid mixture. The term "hybridising specifically" relates to a perfect match between a region of the analyte, e.g. the ZCc of the amplified product, and the capture probe on the microarray. Hybridising specifically takes the length, G/C content and hybridisation conditions, such as salt and temperature, into account as known by the person skilled in the art.

Accordingly, the present invention relates to a method as described herein, wherein said capture probe is located on a microarray. The capture probe is spatially addressable on the microarray.

The microarrays of the present invention may be of any desired size, from two spots to 10⁶ spots or even more. The upper and lower limits on the size of the substrate are determined solely by the practical considerations of working with extremely small or large substrates.

For a given substrate size, the upper limit is determined only by the ability to create and detect the spots in the microarray. The preferred number of spots on a microarray generally depends on the particular use to which the microarray is to be put. For example, sequencing by hybridisation will generally require large arrays, while mutation detection may require only a small array. In general, microarrays contain from 2 to about 10⁶ spots, or from about 4 to about 10⁵ spots, or from about 8 to about 10⁴ spots, or between about 10 and about 2000 spots, or from about 20 to about 200 spots.

Furthermore, not all spots on the microarray need to be unique. Indeed, in many applications, redundancies in the spots are desirable for the purposes of acting as internal controls.

A variety of techniques have been described for synthesizing and/or immobilizing arrays of polynucleotides, including *in situ* synthesis, where the polynucleotides are synthesized directly on the surface of the substrate (see, e.g., U.S. Pat. No. 5,744,305 to Fodor, *et al.*) and attachment of pre-synthesized polynucleotides to the surface of a substrate at discrete locations (see, e.g., WO 98/31836). Additional methods are described in WO 98/31836 at pages 41-45 and 47-48, among other places. The present invention is suitable for use with any of these currently available, or later developed, techniques.

Immobilization of pre-synthesized polynucleotides at different spatial addresses yields an array of polynucleotides whose sequences are identifiable by their spatial addresses.

In embodiments involving *in situ* synthesis of polynucleotides, the polynucleotides are synthesized in their usual manner. The synthetic scheme yields an array of polynucleotides whose sequences are identifiable by their spatial addresses.

The nature and geometry of the solid substrate will depend upon a variety of factors, including, among others, the type of array (e.g., one-dimensional, two-dimensional or three-dimensional) and the mode of attachment (e.g., covalent or non-covalent). Generally, the substrate can be composed of any material which will permit immobilization of the capture probe, e.g. polynucleotide, and which will not melt or otherwise substantially degrade under the conditions used to bind the capture probe, e.g. hybridise and/or denature nucleic acids. In addition, where covalent immobilization is contemplated, the substrate should be activated with reactive groups capable of forming a covalent bond with the capture probe to be immobilized.

Other exemplary suitable materials for use as substrates in the present invention include metal oxides. Metal oxides provide a substrate having both a high channel density and a high porosity, allowing high density arrays comprising different first binding substances per unit of the surface for sample application. In addition, metal oxides are highly transparent for visible light. Metal oxides are relatively cheap substrates that do not require the use of any typical microfabrication technology and, that offers an improved control over the liquid distribution over the surface of the substrate, such as electrochemically manufactured metal oxide membrane. Metal oxide membranes having through-going, oriented channels can be manufactured through electrochemical etching of a metal sheet. Metal oxides considered are, among others, oxides of tantalum, titanium, and aluminium, as well as alloys of two or more metal oxides and doped metal oxides and alloys containing metal oxides. The metal oxide membranes are transparent, especially if wet, which allows for assays using various optical techniques. Such membranes have oriented through-going channels with well controlled diameter and useful chemical surface properties. Patent application EP-A-0 975 427 is exemplary in this respect, and is specifically incorporated in the present invention.

Accordingly, the present invention relates to a method as described herein, wherein said microarray is a flow-through microarray. Also, the present invention relates to a method as described herein, wherein said substrate is a porous substrate, said substrate may be an electrochemically manufactured metal oxide membrane. Preferably, said substrate comprises aluminium oxide.

Accordingly, the present invention relates to a method as described herein, wherein said microarray is a PamChip®.

The composition of the immobilized capture probes is not critical. The only requirement is that they be capable of hybridising to a target nucleic acid of complementary sequence, e.g. the amplified nucleic acid, if any. For example, the capture probes may be composed of all natural or all synthetic nucleotide bases, or a combination of both. Non-limiting examples of modified bases suitable for use with the instant invention are described, for example, in Practical Handbook of Biochemistry and Molecular Biology, G. Fasman, Ed., CRC Press, 1989, pp. 385-392. While in most instances the polynucleotides will be composed entirely of the natural bases (A, C, G, T or U), in certain circumstances the use of synthetic bases may be preferred.

Moreover, while the backbones of the capture probes will typically be composed entirely of "native" phosphodiester linkages, they may contain one or more modified linkages, such as one or more phosphorothioate, phosphoramidite or other modified linkages. As a specific example, one or more immobilized polynucleotides may be a peptide nucleic acid (PNA), which contains amide interlinkages. Additional examples of modified bases and backbones that can be used in conjunction with the invention, as well as methods for their synthesis can be found, for example, in Uhlman & Peyman, 1990, Chemical Review 90(4):544-584; Goodchild, 1990, Bioconjugate Chem. 1(3):165-186; Egholm *et al*., 1992, J. Am. Chem. Soc. 114:1895-1897; Gryaznov *et al*., J. Am. Chem. Soc. 116:3143-3144, as well as the references cited in all of the above.

As such, the capture probes may include polymers of ribonucleotides and deoxyribonucleotides, with the ribonucleotide and/or deoxy-ribonucleotides being connected together via 5' to 3' linkages. The capture probes of the invention may be ribonucleic acids, for example sense or antisense ribonucleic acids, full-length or partial fragments of cRNA, full-length or partial fragments of mRNA, and/or ribo-oligonucleotides. Alternatively, capture probes of the invention may be deoxy-ribonucleic acids, preferably single-stranded full-length or fragments of sequences encoding the corresponding mRNAs. The form of the capture probes should be chosen so that they are complimentary to and form appropriate Watson-Crick hydrogen bonds with the amplified target nucleic acid and/or ligated probes in a sample.

As mentioned above, the capture probes may be polymers of synthetic nucleotide analogs. Such capture probes may be utilised in certain embodiments because of their superior stability under assay conditions. Modifications in the native structure, including alterations in the backbone, sugars or heterocyclic bases, have been shown to increase intracellular stability and binding affinity. Among useful changes in the backbone chemistry are phosphorothioates; phosphoro-dithioates, where both of the non-bridging oxygens are substituted with sulfur; phosphoroamidites; alkyl phosphotriesters and boranophosphates. A-chiral phosphate derivatives include 3'-O-5'-S-phosphorothioate, 3'-S-5'-O-phosphorothioate, 3'-CH₂-5'-O-phosphonate and 3'-NH-5'-O-phosphoroamidate. Peptide nucleic acids replace the entire ribose phosphodiester backbone with a peptide linkage. Locked nucleic acids give additional conformational stability of sugar moiety due to additional bonds between 2'-carboxyl and 5' carboxyl or 4'-carboxyl groups of deoxyribose. Sugar modifications are also used to enhance stability and affinity. The a-anomer of deoxyribose may be used, where the base is inverted with respect to the natural p-anomer. The 2'-OH of the ribose sugar may be altered to form 2'-O-methyl or 2'-O-allyl sugars, which provides resistance to degradation without comprising affinity. Modification of the heterocyclic bases that find use in the method of the invention are those capable of appropriate base pairing. Some useful substitutions include deoxyuridine for deoxythymidine; 5-methyl-2'-deoxycytidine and 5-bromo-2'-deoxycytidine for deoxycytidine. 5-propynyl-2'-deoxyuridine and 5-propynyl-2'-deoxycytidine have been shown to increase affinity and biological activity when substituted for deoxythymidine and deoxycytidine, respectively.

Examples of non-naturally occurring bases that are capable of forming base-pairing relationships include, but are not limited to, aza- and deaza-pyrimidine analogues, aza- and deaza-purine analogues, and other heterocyclic base analogues, wherein one or more of the carbon and nitrogen atoms of the purine and pyrimidine rings have been substituted by heteroatoms, e.g., oxygen, sulfur, selenium, phosphorus, and the like.

The immobilized capture probes may be as few as four, or as many as hundreds, or even more, nucleotides in length. Contemplated as capture probes according to the invention are nucleic acids that are typically referred to in the art as oligonucleotides and also those referred to as nucleic acids. Thus, the arrays of the present invention are useful not only in applications where amplified target nucleic acids or ligated probes are hybridised to immobilized arrays of relatively short (such as, for example, having a length of approximately 6, 8, 10, 20, 40, 60, 80, or 100 nucleotides) capture probes, but also in applications where relatively short capture probes are hybridised to arrays of immobilized target nucleic acids. The capture probes of the array can be of any desired sequence.

In a further embodiment, the microarray of the invention comprises a capture probe comprising the Zipcode sequence which is essentially complementary to a corresponding ZipComcode (ZCc). The capture probe comprising the Zipcode sequence may be spotted or synthesized on a specified location on the microarray. The Zipcode sequence is a unique identifier sequence, which is complementary to the ZipComcode sequence of the probe, which was used to amplify the nucleic acid. The present invention relates particularly to microarrays and the use thereof, comprising unique 20 to 30 base oligonucleotides, for instance 20, 21, 22, 23, 24, 25, 26, 27, 28, 29 or 30 base oligonucleotides, named Zipcodes that are coupled to a porous three dimensional substrate at known locations, as described by van Beuningen *et al.,* (2001, Clinical Chemistry **47**: 1931-1933). These Zipcodes hybridise specifically to molecules containing sequences that are complementary to the Zipcodes, i.e. the ZipComcodes. By linking these ZipComcodes to fluorescent primers via a ligation-amplification reaction, Zipcode microarrays may be used to detect and identify micro-organisms, such as for example microbial specimens. Because the Zipcodes represent unique artificial sequences, microarrays comprising Zipcodes can be used as a universal platform for molecular recognition simply by changing the gene specific sequences linked to the ZipComcodes.

The detection of label on a specified location on microarray, such as the Pamchip indicates the presence of a hybridisation product between the ligated product and the Zipcode sequence on the microarray.

Accordingly, the present invention relates to a method as described herein, wherein said capture probe hybridises specifically to a corresponding ZipComcode. The amplified target nucleic acid or nucleic acids hybridised to a corresponding capture probe or probes on a microarray may result in a hybridisation pattern. The hybridisation pattern, including the intensity of hybridisation, may be characteristic for a given micro-organism.

It will be apparent that the present invention relates to a method as described herein, wherein a signal is detected after hybridising the specifically amplified nucleic acids or the ligated probes to the capture probe. The said signal is preferably a fluorescent or phosphorescent signal, and said fluorescent or phosphorescent signal may be detected by a CCD camera or by laser scanning, such as for example an FD10 system® (Olympus) or a Pamalyzer® (PamGene NV).

As already mentioned before, the price of a microarray presents the larger cost per test. In order to decrease the price per test, the microarray can be interrogated simultaneously with more than one sample. As such, it is contemplated that each individual sample is subjected to the method of the present invention until the hybridisation step, i.e. from each individual sample, the micro-organisms are captured (step a), after which the nucleic acids are extracted (step b), which subsequently undergo a ligase detection reaction. Next, the amplified target nucleic acids derived from all the samples tested (step c) are collectively hybridised to the capture probes on a single microarray (step d) and the hybridised target nucleic acids are detected (step e). The probes pair used per sample may be identical, e.g. detecting the same target nucleic acid, or may differ per sample, e.g. detecting different target nucleic acids. However, in order to differentiate between amplified target nucleic acids from different samples or between different amplified target nucleic acids derived from a single sample, each probe, and thus the amplified target nucleic acid, must be individually assignable and detectable. Hence, each probe comprises a distinct and individually identifiable tag, such as a particular ZipComcode, complementary to a distinct capture probe on the microarray. Although the nucleic acid probe pairs may detect the same target nucleic acids in different samples, each amplified target nucleic acid derived from each sample is traceable because of its discrete tag, corresponding to a specific address on the microarray. In an alternative embodiment, the probes do not comprise tags, but only the primers used for amplification comprise a distinct and individually identifiable tag, such as a ZCc. Obviously, the same considerations as mentioned above apply, in that the tags should differ per sample, and/or per probe, making each individual sample and/or probe identifiable. Accordingly, the present invention relates to a method as described herein, wherein amplified target nucleic acids derived from at least two samples are hybridised to capture probes present on a single microarray.

The data obtained by the methods of the present invention may be further analysed, possibly in an automated fashion. For instance, the hybridisation pattern obtained may be compared to hybridisation patterns stored in a databank. In this regard, the present invention relates also to a computer program stored on computer readable medium capable of performing the comparison of the obtained hybridisation pattern with the hybridisation patterns stored in a databank. Accordingly, the present invention relates to a computer comprising a computer readable medium capable of performing the methods described above. Also, the present invention relates to a computer readable medium comprising a computer program according capable of performing the method described above. Furthermore, the present invention relates to a computer program capable of displaying a web page on a remote computer enabling the use of the method described before.

In a further embodiment, the present invention relates to kits for determining the presence of micro-organisms in a sample comprising the essentials of the methods of the present inventions, for instance, said kits may comprise a filter, possibly means for extracting nucleic acids from said micro-organisms, means for specifically amplifying said nucleic acids, possibly means for analysing the amplified nucleic acids, e.g. microarrays, such as flow through microarrays, possibly buffers and/or an instruction manual.

The characterisation of mixed microbial populations is not easily achieved by current methods and has wide potential application. In most environments where bacteria are found a complex mixture of species is present which may change as a response to local conditions or evolve time. Classical microbiological methods are generally not well suited to the study of these systems as they rely on culture and subsequent isolation of individual colonies. This may only recover a proportion of the species present and also results in large numbers of isolates, which must be characterised. Molecular methods involving the amplification of conserved genes from complex populations and their subsequent characterisation, by cloning and sequencing, or hybridisation, provide alternatives to culture but remain complex. Additionally the amplification step may also introduce bias.

In actively growing populations of cells, each cell contains many copies of the ribosomal . RNAs the specific sequence of which are widely used to identify bacterial species (Woese 1987, Microbiol. Rev. 51:221-271). Due to this natural "amplification" of these sequences within active cells it is possible to detect these sequences without amplification (Small J. *et al*. 2001 App. Environ. Micro. **67**:4708-4716,). Here a method is presented for the extraction and direct identification of ribosomal RNA on a three dimensional array surface. This potentially allows the rapid parallel identification of a wide range of species in a sample without the need for enzymatic amplification or labelling. The method presented here demonstrates almost real time monitoring of complex bacterial communities will be possible which will have application in many areas.

Hence, it will be appreciated that the present invention relates to the methods described above, wherein said step of analysing comprises hybridising a stacking probe to the nucleic acids, nucleic acid mixture and/or cDNA, said stacking probe being complementary for a region of 16S, 18S, 23S or 28S rRNA, thereby providing a nucleic acid/stacking probe complex. Said step of analysing may further comprise hybridising said nucleic acid/stacking probe complex to a capture probe, said capture probe being complementary to a region of the nucleic acid different from the nucleic acid/stacking probe complex. Said capture probe may be specific for a micro-organism. The stacking probe may be labelled. The region of 16S, 18S, 23S or 28S rRNA may be conserved (over species).

It will be evident to the person skilled in the art that the present invention relates to the use of a filter and a microarray as mentioned herein in the method of the present invention. Thus, the present invention relates to the use of at least one pair of a first nucleic acid probe and a second nucleic acid probe as defined supra, including coupled probes, the use of a filter as described above, and the use of at least one set of two primers as defined above, in the methods according to the invention.

Before the subject invention is described further, it is to be understood that the invention is not limited to the particular embodiments of the invention described herein, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

The following examples and figures are offered by way of illustration and not by way of limitation. Nevertheless, the content of said examples and figures may be generalised in the concept of the present invention.

### Short description of the figures

**Figure 1** Sequence alignment of *Staphylococcal* species. The *Staphylococcal* probes comprising a T-tail are bound to the microarray (chip). The stacking probe, comprising a sequence common to *S. aureus, S. epidermidis* and *S. sapprophiticus* is depicted, containing a label at its 3' end.
**Figure 2** Hybridisation signal of different *Staphylococcal* species on PamChip at 55 °C.
**Figure 3** Hybridisation signal of different *Staphylococcal* species on PamChip at 65 °C.
**Figure 4** Ligase Detection Reaction followed by PCR Amplification.
   A: probe comprising two regions cSeq1 and cSeq2 specifically hybridising to target region, two primer binding regions cMse and Eco, and a ZipComcode (ZIP).
   B1: for strain 1, specifically hybridising of probe with cSeq1 and cSeq2 to their complementary sequences Seq 1 and Seq2 of the target region, respectively, after which the single stranded (ss) nick is ligated. for strain 2, hybridising of probe with cSeq1 and cSeq2 to their complementary sequences Seq 1 and Seq2 of the target region, respectively. However, due to a mutation at the 3' site of cSeq2, there is a mismatch between cSeq2 and Seq2 of the target region. Thus, the single stranded (ss) nick cannot be ligated.
   B2: for strain 1, ligation of the ss nick results in a circular probe. for strain 2, the probe remains linear.
   B3: for strain 1, hybridisation with a primer complementary to Mse enables extension of this primer, e.g. via PCR, resulting in a template with the following order of regions, 5'-Mse-Seq1-Seq2-cZIP-cEco-3', and complementary to the probe of A. for strain 2, hybridisation with a primer complementary to Mse enables limited extension of this primer, e.g. via PCR, resulting in a shortened template complementary to the probe of A and with the following order of regions, 5'-Mse-Seq1-3', thus missing the regions Seq2, cZIP and cEco.
   B4: for strain 1, labelled primer Eco, complementary to the region cEco of the template of step B3, is extended. Steps B3 and B4 can be repeated, resulting in exponential amplification. The labelled extension product can be detected, such as for instance by hybridising said product with its ZipComcode (ZIP) region to a complementary capture probe on a microarray.
      for strain2, labelled primer Eco cannot hybridise to a template, and be extended. Consequently, only a linear amplification with the Mse primer is possible.

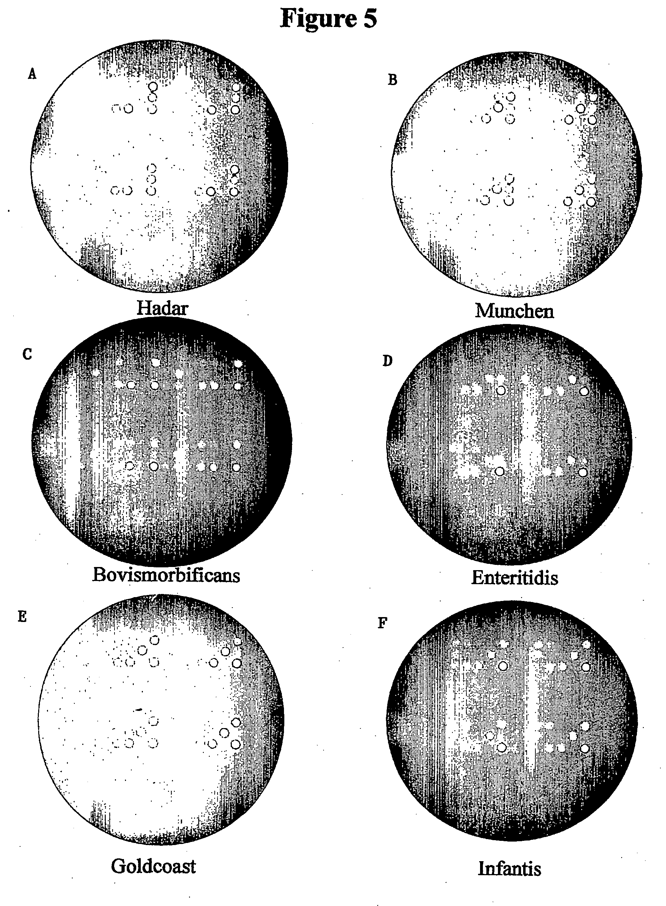

**Figure 5** Comparison of detection products of various *S. enterica ssp enterica* serovars.

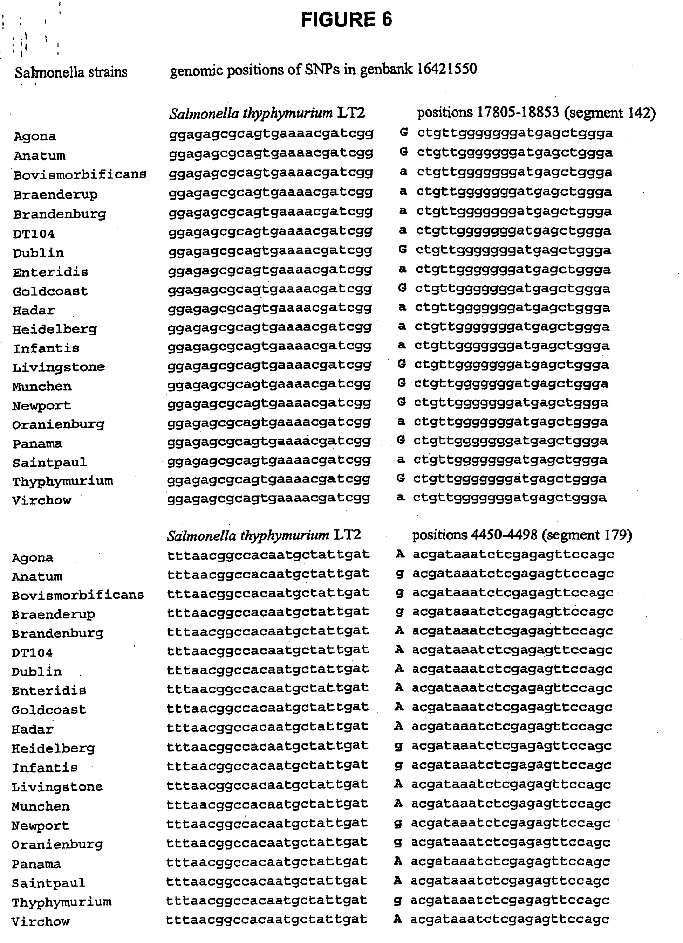

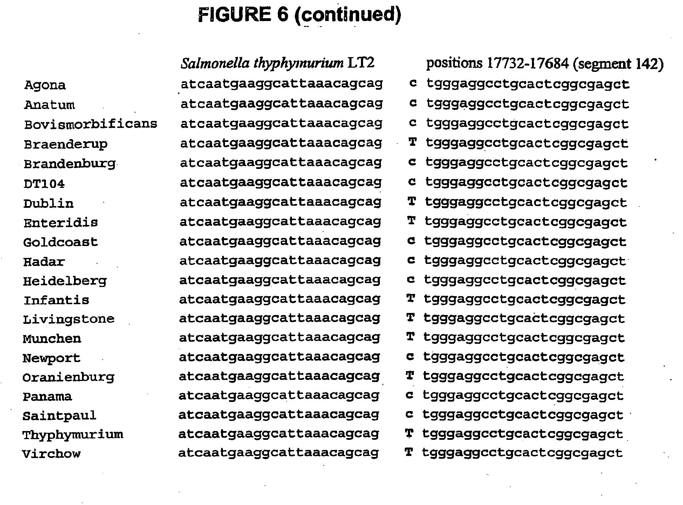

**Figure 6** List of exemplary probe regions for various *S.enterica ssp enterica* serovars.

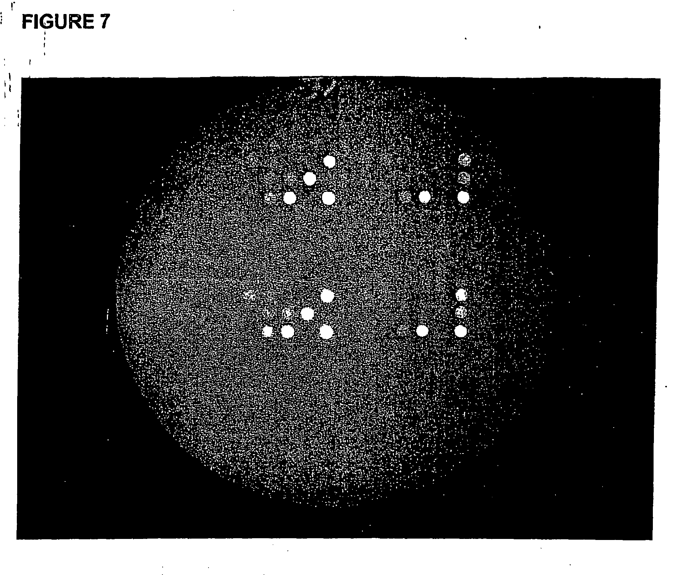

**Figure 7** Comparison of the *S. enterica ssp enterica* serovar patterns detected in milk powder (left hand side) and chicken meat (right hand side), respectively, using a single PAM-chip.

### Examples

### Example 1: Accumulation of micro-organisms from liquids using microsieves

The Micro Analytical Screen (MAS) method of the present invention was elaborated and the performance of Aquamarijn microsieves on laboratory scale and three different pilot plants was examined.
1 In a dairy plant, depending of the dimensions of milk particles, a microsieve with a pore diameter of about 0.5 - 1.2 micron is applied. At a pressure of 10 mbar an averaged flux is measured of 2000 l/m²h (litres per m² membrane surface area per hour). When the process of the present method is applied in the removal of micro-organisms in milk, the largest part of the casein in milk passes through the microsieve membrane and enters the permeate. In experiments with "spiked" skim milk (inoculated with different numbers of colony forming units (cfu's) of *Bacillus subtilis)* with a 0.5 micron microsieve we were able to collect and detect 1 cfu of *Bacillus subtilis* per litre milk.
2 In a beverages plant, depending of the nature of the drinks, a microsieve with a pore diameter of about 0.5 - 1.2 micron is applied. At a pressure of 10 mbar an averaged flux is measured of 3500 l/m²h (litres per m² membrane surface area per hour). When these microsieves were applied, we were able to remove spoilage micro-organisms in beverages. In experiments with "spiked" beverages/drinks (inoculated with different numbers of colony forming units (cfu's) of *Aspergillus niger*) with a 0.8 micron microsieve we were able to collect and detect 1 cfu of *Aspergillus niger* per litre juice.
3 In a beer brewery, depending of the nature of the beers, a microsieve with a pore diameter of about 0.5 - 1.2 micron is applied. At a pressure of 20 mbar an averaged flux is measured of 2500 l/m²h (litres per m² membrane surface area per hour). When these microsieves were applied, we were able to remove spoilage micro-organisms in beverages. In experiments with "spiked" beer (beer inoculated with different numbers of colony forming units (cfu's) of *Kluyveromyces lactis*) and with a 0.8 micron microsieve, we were able to collect and detect 1 cfu of *Kluyveromyces lactis* per litre beer.

When new analysis techniques, e.g. MAS method, are being introduced it is important to validate these technique viz. à viz. commonly used techniques. Therefore, the MAS method is being compared with the "plate count" (PC) method (JECFA).

In our experiments, samples with different yeast cell concentrations have been analysed using both methods. The results obtained show that with the MAS technique, a higher number of yeast cells were counted than with the PC method. This can be explained by the fact that with the MAS technique all individual cells can be counted that form a colony, whereas in the PC method these cells together would count as one single cell after cultivation. In the MAS detection also less viable cells are being counted that would not have been able to multiply on agar media and thus are not found in the "plate count" method.

The collected micro-organisms using the MAS technology can be further characterised and identified by applying microbial molecular biology tools, see below Examples.

### Example 2: Isolation of nucleic acids from samples

### RNA isolation

According to the need, the most appropriate procedure to isolate RNA is chosen. We are using and comparing various procedures to isolate total RNA from micro-organisms. The traditional procedure is the direct injection of bacterial samples into a hot phenol solution (Selinger et al., 2000, Nature Biotechnol. 18, 1262-1268). Alternatively, cells are quickly frozen in liquid nitrogen and mechanically broken before isolation with acid phenol solution.

The cells of Gram-positive organisms are frozen in dry-ice, thawed and sonicated 3 times for 10 sec with a microtip sonicator. The power is set at about 30W. Lysis is indicated by a clear cell suspension.

The methods described above are convenient for isolating RNA.

Subsequently, we have tested the RNAlater® solution (Ambion and Qiagen). In particular, we have examined the permeability of RNAlater® for different microbial species, including *Saccharomyces, Lactococcus, Leuconostoc* and *Lactobacillus.* For these four species the obtained results ranged from sufficient to good.

In addition, a variety of RNA isolation kits, available from different commercial sources (Ambion, Qiagen, Sigma-Aldrich and others) are tested, and qualified as convenient.

### DNA isolation

The method to lyse the micro-organism varies depending on the type of micro-organism.

After removing the micro-organisms from the surface of the microsieve, the cells were suspended by vortexing for at least 20 sec., in 300-500 µl of sterile distilled water. The DNA was extracted after cell lysis by immersing the tubes in boiling water for 10 min. The cell debris was pelleted by centrifugation (13 krpm for 10 sec.) and the supernatant containing the DNA was removed and transferred to a fresh tube.

An alternative method was based on the lysing and nuclease inactivating properties of the chaotropic agent guanidinum thiocyanate (GuSCN), and the nucleic acid-binding properties of silica particles or diatoms in the presence of this agent as described by Boom *et al.* (Boom *et al*., 1990, J.Clin. Microbiol **28**:495-503).

In case of Gram positive organisms, including *Lactobacillus,* the cells were suspended in 500 µl of STE buffer (100mM NaCl, 50mM Tris-HCl, 10mM sodium EDTA, pH 7.5) and incubated at 37°C with 100 µl of lysozyme (10mg/ml) for 15 minutes. The lysozyme treated cells were further processed according the instructions of the QIAgen DNeasy Tissue kit (Westburg, Leusden, the Netherlands).

In case of Gram negative organisms, including *Salmonella,* genomic DNAs from the samples were extracted and purified using Genomic tips (Qiagen) with Genomic DNA buffer set (Qiagen), or the Wizard^{™} genomic DNA purification kit (Promega).

In case of moulds or fungi like *Aspergillus* the cells were suspended in 500 µl OM (Osmotic Medium: 1.2 M MgSO₄, 8.4 mM Na₂HPO₄, 1.6 mM NaH₂PO₄, pH 5,8), and incubated at 30 °C with 50 µl Glucanex® (Novozymes, Denmark), for 15-30 minutes. The pectinolytic treated cells were further processed according the instructions of the QIA DNA miniprep kit, or the Puregene genomic DNA isolation kit (Gentra).

The concentration of the DNA isolated according to the various procedures was estimated by spectrophotometry at 260 nm (Gene Quant II RNA/DNA calculator®, Amersham Pharmacia Biotech, Woerden, the Netherlands).

### Example 3: Design of an universal capture DNA microarray

We have designed a set of 20⁴ (20-mer) Zipcode DNA sequences (Gerry *et al*., 1999, J.Mol.Biol. **292**: 251-262). All these Zipcodes have been blasted with help of:
- TIGR microbial database: www.tigr.org/tdb/mdb/mdbcomplete.html.or with
- Genbank ; www.ncbi.nlm.nih.gov.
- IECB University of Vienna: www.probebase.net.

In addition, all Zipcodes showing internal or external homologies, all Zipcodes falling outside the desired Tm range (60 - 72 °C) and Zipcodes which are difficult to synthesize e.g. a track of 5 or more dGTPs were excluded from further analysis.

The remaining 180 Zipcodes were synthesized and provided by the supplier Proligo (Paris, France), Eurogentec (Liege, Belgium), or Metabion (Martinsried, Germany).

Microarrays were prepared using PAM chips designed to covalently immobilize modified oligonucleotides as described in patent WO 99/02266.

Spotting the 180 modified Zipcode oligonucleotides (20-mers) was performed using a non-contact piezo driven dispensing system (BioChip arrayer Packard, Perkin Elmer).

Also positive and negative control oligonucleotides were spotted on the Zipcodes containing microarray.

From the selected 180 Zipcodes spotted on the microarray, the complementary sequences, i.e. the oligonucleotides comprising the ZipComcode, were obtained from the suppliers Proligo (Paris, France), Eurogentec (Liege, Belgium), or Metabion (Martinsried, Germany).

These ZipComcode oligonucleotides were used in SNPWave^{™} reactions as described in Example 4.

The results obtained with this universal capture DNA microarray are presented in Example 6.

### Example 4: Multiplexed amplification and labelling of the isolated target sequences/ nucleic acids

The probes for LDR were designed to be specific to the rDNA. Probes were designed to be specific for the 16S or 23S sequences of the bacterial groups under investigation, as well as for the 18S or 28S sequences of the yeast and fungal groups under investigation. For each of these groups, a substantial number of rDNA sequences were evaluated, assembled in sub-groups and aligned using the Clustal W Algorithm. This yielded a consensus sequence for each group with a cut off of 75 % (meaning that 3 out of 4 sequences determined had the consensus sequence at a given position).

In this case, a common probe II was used, i.e. common to all bacterial groups, yeast groups or fungal groups under investigation. The specific identification was accomplished by the discriminating probe I. The specificity of each set of probe pairs was scrutinised with the Probe Match tool, to ensure that no cross-hybridisation occurs between probes and between probes and target sequences.

All primers were designed to have melting temperature (Tm) values with the corresponding probes of between 60 and 72 °C. The discriminating primers I (comprising a sequence identical to PBS I) were purchased with a Cy3 molecule at their 5' terminal position, while the common primers II (comprising a sequence complementary to PBS II) comprising a ZipComcode and a phosphate at their 5' terminal position.

The LDR reaction was carried out in a final volume of 20 µl containing 20 mM KCl, 10 mM MgCl₂, 20mM Tris-HCl (pH 7.5), 0.1% NP40, 0.01 mM ATP, 1 mM DTT, 2 pmol of each discriminating probe I, 2 pmol of each common probe II and 1 - 500 fmol of the isolated DNA product/target sequences (see example 2).

This reaction mixture was preheated for 2 min at 94 °C and centrifuged in an Eppendorf micro-centrifuge for 20 sec, then 1 µl of 4 U/µl *Pfu* DNA ligase (Stratagene, La Jolla, California) was added. The LDR was cycled for 40 rounds of 94 °C for 30 sec and 64°C for 4 min. in a PCR Express thermal cycler (Hybaid, United Kingdom).

The LDR reaction products were hybridized with the Pamchip-microarray where the Zipcode sequences, that are complementary to the ZipComcodes, have been spotted.

### Example 5: Conversion of AFLP fragments into species-specific "signature" sequences

From the following micro-organisms, see Table 1, genomic DNA was isolated and purified using the Qiagen genomic kit (Westburg, Leusden, the Netherlands). Primary template DNA was prepared using the restriction enzymes EcoRl and *Mse*1.

AFLP analysis was performed as described by Vos *et al*., *supra,* except that the streptavidin bead selection was omitted. PCRs were performed using primer pairs derived from each of two sets of primers. Primers in the EcoRI set all included the core sequence E:
5' - GACTGCGTACCAATTC- 3') [SEQ ID NO: 1] with 1 or 3 basepair extensions.
Primers of the Msel set have the sequence M:
5' - GATGAGTCCTGAGTAA - 3' [SEQ ID NO: 2] with 1 or 3 basepair extensions. The primer combinations (E_{A} and M_{C}) and (E_{C} and M_{A}) were used for pre-amplification of the primary microbial template. To reduce the number of AFLP fragments, three selective nucleotides per primer were used to generate AFLP fragments from the secondary templates. AFLP bands were labeled with a radioactive probe according the manufacturer's instructions (Amersham Pharmacia). The labeled AFLP bands were separated by electrophoresis on 6 % (w/v) polyacrylamide denaturing sequencing gels and visualized by exposing X-ray film to the dried gel. AFLP fragment isolation and cloning target AFLP bands on the autoradiograph were matched to the corresponding area in the gel and the appropriate AFLP fragments in the range of 20 through 250 nucleotides were excised from the dried gel. The bands were eluted from the gel by incubation in 100 µl of distilled water at 4 °C for 1 h.

For each species, 5 bands in the range of 20 through 250 nucleotides were randomly chosen, isolated as described for the AFLP bands, amplified and cloned into a suitable PCR vector e.g. pGEM-T (Promega, Leiden, The Netherlands) or pCR2,1-TOPO (Invitrogen, Carlsbad, Ca, USA).

After cloning in the *Escherichia coli* K12 host, 5 colonies of each selected transformed band were sequenced using the Sequenase version 2.0 DNA sequencing kit (United States Biochemical, Cleveland, Ohio, USA). Determination of the DNA sequence of the *E.coli* K12 clones allowed PCR primers to be designed for each unique DNA sequence using Oligo 5.0 software (PE Biosystems, Foster City, California, USA). The PCR products were analysed on 4% (w/v) Metaphor agarose gels (FMC, Rockland, Me, USA).

All the obtained DNA sequences from the AFLP^{™} experiments have been blasted with help of the Genbank (www.ncbi.nlm.nih.gov) ,TIGR (www.tigr.org/tdb/mdb/mdbcomplete.html) or IECB University of Vienna: www.probebase.net databases. In addition, all obtained DNA sequences from the AFLP^{™} experiments showing internal or external homologies and falling outside the desired Tm range (50 - 75 °C) were eliminated.

The remaining sequences, which are species specific, were named "signature sequences/tags". Suitable signature sequences/tags in the range of 20-60 nucleotides were synthesized by the supplier (Proligo, Paris, France or Eurogentec, Liege, Belgium, or Metabion, Martinsried, Germany)).

These signature sequences/tags have been used to develop multiplex amplification and/or microarray typing.

Microarrays are prepared using PAM chips, designed to covalently immobilize modified oligonucleotides according to the teaching of WO 95/11755, as described in Example 3.

### Example 6 : Detection and Identification of micro-organisms by combining the AFLP Technology with the Pamgene technology.

The universal Zipcode microarray together with positive and negative control sequences is designed as described in Example 3.

The DNA/RNA samples under investigation are amplified and labelled in the presence of the appropriate primers according the LDR protocol as described in Example 4. Half of the SNPWave^{™} reaction products are analysed on a MegaBACE station. The other half of the SNPWave^{™} reaction products are hybridised to the Zipcode microarray.

### Hybridisation protocol

Incubations are performed in a thermostatically controlled incubator holding one chip, which consists of four microarrays. Each microarray is hybridised to a sample by pulsing back and forth of the target solution through the pores of the microarray substrate using a Microlab 500 syringe pump (Hamilton, Nevada, USA) at a rate of 20 µl per 10 seconds. Real time monitoring of the reaction is possible with an Olympus BX41 or FD10 microscope (Olympus, Tokyo, Japan) with an 8 bit CCD camera (Kappa OptoElectronics GmbH, Germany and associated capture program).

Each array is pre-wetted (by pumping) with 2 washes of 25 µl PBS plus 0.1% Tween 20 (Sigma), then rinsed twice with 25 µl 0.6 x SSC for 30 seconds prior to the hybridisation. Hybridisation and detection is carried out in a volume of 25 µl 0.6 x SSC with continuous pumping of the mixture up and down through the array. The target, either 5 µl PCR product (approximately 0.2 pmol) at 95 °C directly from the PCR cycler or 0.5 pmol of the appropriate target, is added to the hybridisation solution on the array. After addition of the target to the hybridisation buffer on the array, hybridisation is allowed to continue for 20 minutes at a set temperature of 55 °C, to allow the hybridisation to reach equilibrium. A Microsoft Windows bitmap (BMP) image is captured, at the point in the mixing cycle when the hybridisation solution is below the array, and then the temperature is increased at approximately 1 °C every 2 minutes. For each degree increase in temperature, a separate image is captured.

### Image Analyses

images are analysed using ArrayPro Software (Media Cybernetics, Silver Spring, MD, USA). Median signals are calculated using local corners background removal for each spot and data is exported to Excel (Microsoft) for further analysis.

### Example 7 : Method for the direct detection and identification of rRNA on a flow through microarray substrate

### Identification and characterisation of Staphylococcal species.

Any nucleic acid procedure can be used which results in the isolation of the ribosomal RNA. In this instance the Boom method (see above) was used to extract DNA and RNA from a culture of *S. aureus* (289) and *S. epidermidis* (286) after initial lysostaphin enzymatic disruption of the bacterial cell wall. The bacteria were harvested by centrifugation from 0.5 ml of broth, and resuspended in 100 µl of water. A 1 µl of a 1 mg/ml solution of lysostaphin was added and the suspension was incubated for 20 minutes at room temperature.

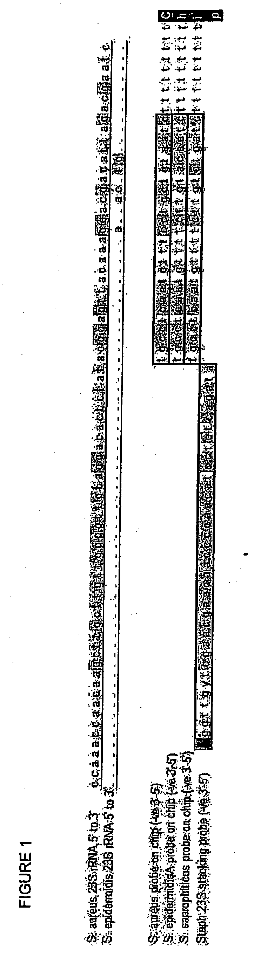

A 2 µl aliquot of this extract was heated for 2 minutes in the presence of 1 µl of 1 µM solution of a labelled fluorescent probe (Staph 23S stacking Probe) complementary for a region of the Staphylococcal 23S rRNA (Figure 1).

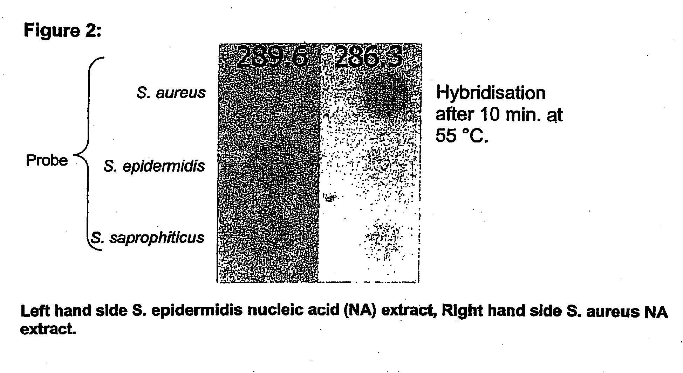

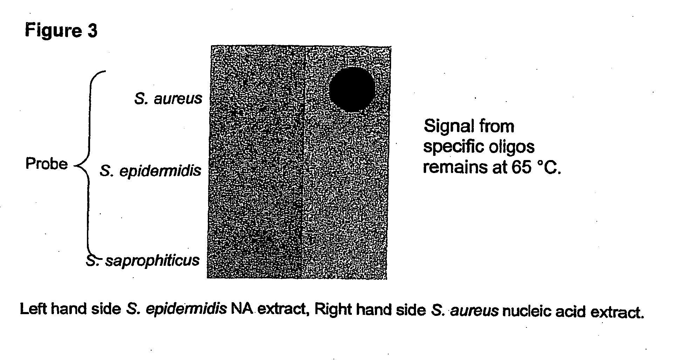

The hot Sample probe mixture was immediately added to a wet PamChip (van Beuningen *et al.* Clin. Chem. 47:1931-1933) spotted with three oligonucleotides specific for different Staphylococcal species (Anthony *et al*. (2000) J.Clin.Microbiol. 38:781-788) containing 25 µl of a 0.6 x SSC solution at 55°C. The chip was monitored under a fluorescent microscope while pumping the hybridisation solution through the PamChip once every 30 seconds and any signal recorded. Signal was detected within 1 minute. The hybridisation was allowed to continue for 10 minutes after which time the signal was very strong (Figure 2). The identity of any sequence detected was confirmed by heating the PamChip to 65°C and monitoring any decrease in the fluorescent signal (Figure 3). A specific signal was detected from the spot corresponding to the species the rRNA was extracted from.

### Conclusion

This experiment demonstrates that the rapid, parallel, direct, and specific, identification of bacteria without the need for enzymatic labelling or amplification is practical.

### Example 8: Description of bacterial strains and DNA isolation

The following 20 *Salmonella* strains were chosen for analysis:
1. *Salmonella enterica ssp. enterica* serovar Agona
2. *Salmonella enterica ssp. enterica* serovar Anatum
3. *Salmonella enterica ssp. enterica* serovar Bovismorbificans
4. *Salmonella enterica ssp. enterica* serovar Braenderup
5. *Salmonella enterica ssp. enterica* serovar Brandenburg
6. *Salmonella enterica ssp. enterica* serovar DT104
7. *Salmonella enterica ssp. enterica* serovar Dublin
8. *Salmonella enterica ssp. enterica* serovar Enteritidis
9. *Salmonella enterica ssp. enterica* serovar Goldcoast
10. *Salmonella enterica ssp. enterica* serovar Hadar
11. *Salmonella enterica ssp. enterica* serovar Heidelberg
12. *Salmonella enterica ssp. enterica* serovar Infantis
13. *Salmonella enterica ssp. enterica* serovar Livingstone
14. *Salmonella enterica ssp. enterica* serovar München
15. *Salmonella enterica ssp. enterica* serovar Newport
16. *Salmonella enterica ssp. enterica* serovar Oraniënburg
17. *Salmonella enterica ssp. enterica* serovar Panama
18. *Salmonella enterica ssp. enterica* serovar Saintpaul
19. *Salmonella enterica ssp. enterica* serovar Thyphimurium
20. *Salmonella enterica ssp. enterica* serovar Virchow

Pure cultures were inoculated into nutrient broth and grown o/n at 37°C. One ml of this o/n culture was used for DNA isolation using the Qiagen genomic DNA isolation kit according to the procedures advised by the manufacturer (Qiagen, Westburg, Leusden, the Netherlands).

### Example 9: AFLP fingerprinting of Salmonella strains

AFLP analysis was performed using the primer combination Nialll-Taql as described by Vos *et al.,* Nucleic Acids Research 23(21), (1995), 4407-4414. PCRs were performed using primer pairs derived from one Taql- and one Nlalll-AFLP primer. AFLP primers for Nlalll all included the following core sequence:
5'- GACTGCGTACACTAG-3' with 2 basepair extensions.
AFLP primers for Taql all included the following core sequence:
5'-GATGAGTCCTGAGCGA-3' with 2 basepair extensions.

Four Nlalll-primers +2 were used in combination with four Taql-primers +2 yielding 16 primer combinations (all possible pairwise combinations). The Nlalll-primers were endlabeled by phosphorylation of the 5'OH with ³³p-ATP as described by Vos *et al*., Nucleic Acids Research 23(21), (1995), 4407-4414. The 16 AFLP primer combinations were used for generating AFLP-fingerprints on all 20 Salmonella serovars, described in "example 8". The AFLP primer combinations used are depicted below.

| Nlalll-primers | Taql-primers |
|---|---|
| Nlalll-AC | Taql-AC |
| Nlalll-AG | Taql-AG |
| Nlalll-TC | Taql-TC |
| Nlalll-TG | Taql-TG |

AFLP fragments were separated by electrophoresis on 6 % (w/v) polyacrylamide denaturing sequencing gels as described by Vos *et al.,* Nucleic Acids Research 23(21), (1995), 4407-4414. After electrophoresis gels were transferred to Whatman 3MM-paper (Whatman plc, Kent, U.K.) and dried on a BIORAD (Biorad inc., Hercules, CA, U.S.A.) slab gel dryer. AFLP-fingerprints were visualized by exposing the dried gel to X-ray film (Eastman Kodak, New Haven, CT, U.S.A.).

### Example 10: Characterization of AFLP-fragments

The autoradiograph images of the AFLP-fingerprints of the *Salmonella* strains from "example 8", were inspected for potentially valuable AFLP-fragments, with emphasis on AFLP-fragments differing between the various strains. A total of 50 potentially interesting fragments were cut out from the corresponding dried gel, and rehydrated for 2 hours in 50 *µ*l of 10 mM Tris.HCl; 1 mM EDTA pH 8.0. In this way the AFLP-fragments will elute into the aqueous solution. Next, 10 *µ*l of each of the AFLP-eluates was used to generate recombinant clones in *E.coli* using the "HTP Zero Blunt TOPO PCR Cloning Hit for Sequencing" according to the instructions provided by the manufacturer Invitrogen (Invitrogen Corp., Carlsbad, CA, U.S.A.). Plasmid DNAs were isolated from 2 clones of each of the 50 cloning events using the alkaline plasmid DNA isolation method of Bimboim & Doly (Nucleic Acids Research 7[6], 1979, 1513-1523) yielding 100 plasmid DNA preparations. The AFLP-fragment inserts of each of the 100 plasmid DNAs were sequenced on a MegaBACE 1000 capillary DNA sequencer (Amersham Biosciences, Piscataway, NJ, U.S.A.) using the "DYEnamic ET Dye Terminator Kit (Amersham Biosciences, Piscataway, NJ, U.S.A.).

### Example 11: Matching of the AFLP fragment to the Salmonella thyphymurium LT2 whole genome sequence

The sequence of the 50 AFLP-fragments were matched to the complete genome sequence of *Salmonella thyphymurium* LT2 (McCleland et al., Nature 413, [2001], 852-856; genbank accession number 16421550). All fragments could be traced back to the genome sequence, and 36 corresponding genomic segments evenly spread over the whole genome were selected for further analysis.

### Example 12: PCR amplification and sequencing of genomic DNA fragments of Salmonella strains

The software package OLIGO-6 (MedProbe, Oslo, Norway) was used to select PCR-primer sets for amplification of the 36 genomic DNA segments of "example 11" directly from genomic DNA preparations. Primer sets were selected to amplify the sequence encompassing the original AFLP-fragments and in addition at least 50 base-pairs up- and downstream sequences. Furthermore, the OLIGO-6 package was used to select sequencing primers for direct sequencing of the PCR-products of all 36 PCR primer pairs in two directions. Amplification reactions with all 36 primer pairs on 4 selected *Salmonella* serovars (example 8) were carried out using the same PCR-conditions as for the AFLP analysis of "example 9", except that 10 pMol of each primer was used in a total of 50 µl reaction volume. Twenty-seven of the 36 primer pairs gave a good and uniform PCR-product on these 4 genomic *Salmonella* DNAs. These 27 primer pairs were subsequently used to generate PCR-products on all 20 *Salmonella* serovars of "example 8".

PCR fragments were sequenced on a MegaBACE 1000 capillary DNA sequencer (Amersham Biosciences, Piscataway, NJ, U.S.A.) using the "DYEnamic ET Dye Terminator Kit (Amersham Biosciences, Piscataway, NJ, U.S.A.). Fragments were sequenced in two directions using the sequencing primers selected by the OLIGO-6 software package.

### Example 13: Identification and selection of Single Nucleotide Polymorphisms

For each of the 27 genomic segments, the sequences of all 20 PCR-products were aligned using the ClustalW software package (freely available from the European Bioinformatics Institute, www.ebi.org, Hinxton, U.K.). An example of 3 of such multiple sequence alignments is depicted in Figure 6. In the 27 segments a total of 222 positions were identified having a potential Single Nucleotide Polymorphism (SNP). An SNP is defined in this context as a nucleotide position were at least one base differs from all other bases at that position (Figure 6); in most cases two sequence variants will occur in a certain percentage the individuals tested, which is defined as the allelic frequency. SNPs having an allelic frequency of around 50% are generally the most informative ones. In addition to the allelic frequency, the sequence context surrounding the SNP is an important aspect for SNP-selection. For the design of ligation probes from the SNP-collection, the following major criteria were applied:
- Allelic frequency around 50%;
- No additional SNPs 25 nucleotides up- and downstream of the SNP;
- An average G-C content of 40 - 60% in the 25 nucleotides up- and downstream of the SNP.

### Example 14: Design of ligation-amplification probes

The ligation-amplification probes had the following design (from 5'-3'), with segments a, b, c, d, e, going from the 5'end to the 3'end (Figure 4A):
1. Segment a of 20-30 nucleotides complementary to the target sequence (Figure 4A, cSeq1);
2. Segment b of 19 nucleotides complementary to amplification primer 2 (Figure 4A, cMse);
3. Segment c of 19 nucleotides identical to amplification primer 1 (Figure 4A, Eco);
4. Segment d of 24 nucleotides comprising the ZipComcode-sequence (Figure 4A, ZIP);
5. Segment e of 20-30 nucleotides complementary to the target sequence and located immediately downstream of segment a (Figure 4A, cSeq2)

All probes were ordered at Metabion GmbH (Martinsried, Germany).

### Example 15: ligation-amplification reactions (Figure 4B1 - 4B4)

The amplification primers had the following sequence:
Primer 1 (Eco): 5'-FAM-GTAGACTGCGTACCAATTC-3'
Primer 2 (Mse): 5'-GACGATGAGTCCTGAGTAA-3'
The primers were ordered at Metabion GmbH (Martinsried, Germany); FAM is the name for the fluorescent dye covalently attached to the 5'-end of primer 1, and used to label the amplification products.

The ligation reactions were carried out in a volume of 10 µl containing:
1.0 fMol of each ligation-amplification probe oligonucleotide,
10⁴ molecules of target-DNA of the organisms to be analyzed,
5 units Taq DNA ligase (New England Biolabs, Beverly, MA, U.S.A.),
1.0 µl 10 x Taq DNA ligase buffer (New England Biolabs, Beverly, MA, U.S.A.), sterile water to an end volume of 10 µl.

The ligation reaction was incubated for 30 seconds at 98 °C, and subsequently for 20 hours at 55 °C in a Biorad "iCycler" (Biorad, Hercules, CA, U.S.A.).

After ligation, an exonuclease treatment was carried out to remove all non-reacted probes. For this purpose 10 µl of a solution was added containing 5 units exonuclease I and 5 units exonuclease III in 20 mM Tris.HCl pH 8.5. The reaction was incubated at 30 minutes for 37 °C, and next for 30 minutes at 80 °C.

Finally the amplification reaction was carried out. For this purpose, 30 µl of a solution was added containing 10 pMol of primer 1, 10 pMol of primer 2, 0.5 units Amplitaq DNA polymerase (Applied Biosystems, Foster City, CA, U.S.A.), 0.35 mM dNTPs (from a 25 mM dNTP-mix, Amersham Biosciences, Piscataway, NJ, U.S.A.) in 20 mM Tris.HCl pH 8.5. The PCR was carried out using the amplification conditions as described by Vos *et al.,* Nucleic Acids Research 23(21), (1995), 4407-4414.

### Example 16: Detection of amplification products on the FD10 hybridization station

Incubations are performed in a thermostatically controlled incubator holding one chip, which consists of four microarrays. Each microarray is hybridised to a sample by pulsing back and forth of the target solution through the pores of the microarray substrate using a Microlab 500 syringe pump (Hamilton, Nevada, USA) at a rate of 20 µl per 10 seconds. Real time monitoring of the reaction is possible with an Olympus BX41 or FD10 microscope (Olympus, Tokyo, Japan) with an 8 bit CCD camera (Kappa OptoElectronics GmbH, Germany and associated capture program).

Each array is pre-wetted (by pumping) with 3 washes of 50 µl 0.6 x SSPE (1 x SSPE = 150 mM NaCl, 15 mM NaH₂PO₄, 1 mM EDTA, pH 6.8). Hybridisation and detection is carried out in a volume of 50 µl 0.6 x SSPE containing 5 µl of the reaction products (e.g. of example 15), with continuous pumping of the mixture up and down through the array. The hybridisation is carried out at 55 °C for 10 minutes. A 12 bits Microsoft TIFF image is captured at the end of the 10 minutes hybridisation.

Images are analysed using ArrayPro Software (Media Cybernetics, Silver Spring, MD, USA). Median signals are calculated using local corners background removal for each spot and data is exported to Excel (Microsoft) for further analysis.

### Example 17: ZIP code sequences

Bacteriophage lambda of *E. coli* was used (48,501 base pairs) to select a set of 49 ZIP-sequences. The DNA genome of the bacteriophage was scanned using the software package OLIGO-6 (MedProbe, Oslo, Norway) to select 24-mer sequences with a Tm around 60 °C at 150 mM NaCl,100 pM probe, a GC-content of 40 - 60%, and minimal internal base-pairing. As an example, 10 of such lambda-derived ZIP-sequences are depicted below.

| | |
|---|---|
| ZIP-01 | 5-TACATATCACAACGTGCGTGGAGG-3 |
| ZIP-02 | 5-CCTCATGTCAACGAAGAACAGAAC-3 |
| ZIP-03 | 5-TTATGGTGATCAGTCAACCACCAG-3 |
| ZIP-04 | 5-TCCATGCGCTTGCTCTTCATCTAG-3 |
| ZIP-05 | 5-GCCTTACATACATCTGTCGGTTGT-3 |
| ZIP-06 | 5-CACAAGGAGGTCAGACCAGATTGA-3 |
| ZIP-07 | 5-ACACATACGATTCTGCGAACTTCA-3 |
| ZIP-08 | 5-TTACAGGATGTGCTCAACAGACGT-3 |
| ZIP-09 | 5-GCTCACAATAATTGCATGAGTTGC-3 |
| ZIP-10 | 5-TCACGCACTGACTGACAGACTGCT-3 |

### Example 18: Detection of Salmonella serovars

1. DNA was isolated from the 20 bacterial strains described in example 8;
2. A set of 17 SNPs was selected for discrimination of the various serovars (examples 9, 10, 11, 12 and 13);
3. Ligation-amplification probes were designed as described in example 14, using 17 of the 49 ZIP-sequences (ZCc) from example 17;
4. A ligation-amplification reaction was carried out as described in example 15;
5. A microarray using PAM-chips was manufactured to which 196 oligonucleotides were covalently immobilized complementary in a 14 by 14 format using 4 sets of 49 oligonucleotides: a 7 x 7 array of oligonucleotides (Zipcodes) was spotted in quadruple. Oligonucleotide numbers 2, 3, 4, 5, 6, 7, 9, 10, 11.,12, 13, 14, 15, .16, 17, 18 and 19 were complementary to the 17 ZIPComcode sequences of the ligation-amplification probes of step 3. Oligonucleotides 1, 8, 20 and 21 were used for control purposes.
6. An aliquot of the PCR-products of step 4 were hybridized to microarrays of step 5, as described in example 16;
7. Microarray images were analyzed as described in example 16 (see Figure 5).

Figure 5 depicts the results the PCR-products hybridized to a PAM-chip (in quadruple). The microarray provides a unique pattern for every serovar, enabling detection and identification. The pattern is characterized by the hybridization per se, as well as the intensity of the hybridization.

### Example 19: Detection of Salmonella serovars in multiple food samples

1. Two samples were taken, one from milk powder, and one from chicken meat. Samples of 100 g were taken, that were subsequently transferred into 250 ml of EEB (Enterobacteriaceae Enrichment Broth), and incubated for 16 hours at 37 °C. The chicken meat was ground before transfer to the EEB-medium using a standard meat grinding machine for domestic use;
2. After incubation, one ml of both samples was taken and DNA was isolated from the two samples as described in example 8;
3. A set of 17 SNPs was selected for discrimination of the various serovars as described in example 18.
4. Ligation-amplification probes were designed as described in example 14: 2 sets of each 17 probes (ZIPComcodes) were designed using 34 of the 49 ZIP-sequences from example 17. These probe sets were identical with the exception of the ZIPComcode sequences, that were completely different;
5. Ligation-amplification reactions were carried out as described in example 15; probe set 1 was used for sample one originating from the milk powder, probe set 2 was used for sample two originating from the chicken meat;
6. A microarray using PAM-chips was manufactured as described in example 18. The 17 oligonucleotides (Zipcodes) complementary to the 17 ZIPComcodes of probe set 1 were located in duplicate on the left hand side of the PAM-chip; the 17 oligonucleotides (ZIPcodes) complementary to the 17 ZIPComcodes of probe set 2 were located in duplicate on the right hand side of the PAM-chip;
7. Two aliquots of the PCR-products of step 5 of sample 1 and sample 2 respectively, were mixed and hybridized to microarrays of step 6, as described in example 17;
8. Microarray images were analyzed as described in example 17 (see Figure 7).

| Table 1: Microbial species under investigation. | |
|---|---|
| | |

| GENUS | Species |
|---|---|
| Escherichia | -coli, |
| Salmonella | -typhimurium, -enterica, -enteritidis |
| Shigelia | -boydii, -dysenteriae, -flexneri, -sonnei, |
| Enterobacter | -sakazakii |
| Mycobacterium | -africanum, -fortuitum, -smegmatis, -(para)tuberculosis, -xenopi, |
| Listeria | -monocytogenes, -grayi, -seeligeri, |
| Campylobacter | -coli, -jejuni, -lari, -upsaliensis, |
| Legionella | -pneumophila, -longbeachae, jordanis, -dumoffi, -micdadei, -gormanii |
| Lactobacillus | -acidophilus, -casei, johnsonii, -rhamnosus |
| Lactococcus | -lactis, -cremoris, |
| Bacillus | -cereus, -coagulans, -thurigiensis, -bifermentus |
| Clostridium | -botulinum, -perfringens, -tyrobutyricum, -butyricum, -sporogenes, -sordellii |
| Leuconostoc | -lactis, -mesenteroides, -dextranicum, -cremoris |
| Staphylococcus | -aureus, -epidermidis, -saprophiticus |
| Vibrio | -parahaemolyticus |
| Yersinia | -enterocolitica |
| Streptococcus | -thermophilus, -bovis, -pyogenes |
| Enterococcus | -faecalis, -mundtii, -faecium, -casseliflavus |
| Micrococcus | -luteus, -varians, -roseus, -agilis |
| Pseudomonas | -fluorescens, -putida, -aeruginosa |
| Flavobacterium | -sp. |
| Alcaligenes | -faecalis, -sp. |
| Microbacterium | -lacticum, -sp. |
| Acinetobacter | -calcoaceticus |
| Enterobacteriaceae / Coliforms | |
| | |
| Kluyveromyces | -lactis |
| Saccharomyces | -cerevisiae |
| Candida | -guillermondi, -lusitaniae |
| Hansenula | -anomala |
| Rhodoturula | -rubra |
| Torulopsis | -candida, -maris |
| Trichosporon | -beigelli |
| | |
| Aspergillus | -flavus, -parasiticus, -ochraceus, -niger, -tubigiensis |
| Neurospora | -crassa |
| Geotrichum | -candidum |
| Blakeslea | -trispora |
| Penicillium | -emersonli, -funiculosum, -glaucum, -notatum, -roquefortii, -camembertii |
| Rhizomucor | -miehei, -genevensis, javanicus, -pusillus |
| Rhizopus | -arrhizus, -chinensis, -delamar |
| Trichoderma | -reesei, -viridi |
| | |

## Claims

1. A method for determining the presence of micro-organisms in a sample, comprising the steps of:
(a) capturing of said micro-organisms if present,
(b) extracting nucleic acids from said micro-organisms, said nucleic acids comprising target nucleic acids,
(c) performing a ligase detection reaction (LDR) on said target nucleic acids, comprising:
(c1) providing a pair of a first nucleic acid probe and a second nucleic acid probe, said first nucleic acid probe comprising a 3' located target-specific sequence I complementary to a distinct part of said target nucleic acid and said second nucleic acid probe comprising a 5' located target-specific sequence II complementary to a second part of said target nucleic acid located essentially adjacent to and 3' from said target-specific sequence I, wherein said first nucleic acid probe further comprises a 5' located primer binding section I (PBS(I)) and possibly a stuffer, and said second nucleic acid probe comprises a 3' located primer binding section II (PBS(II)) and possibly a stuffer; and wherein the first nucleic acid probe or the second nucleic acid probe further comprises a region (ZipComcode) which is (i) essentially complementary to a corresponding region of a capture probe on a microarray after amplification and (ii) essentially non-complementary to said target nucleic acid, and (iii) which is located in between the target specific sequence and the primer binding section;
(c2) incubating said target nucleic acid with said first nucleic acid probe and said second nucleic acid probe under conditions allowing hybridisation of complementary nucleic acids,
(c3) connecting any essentially adjacent probes,
(c4) providing at least one set of two primers, wherein the first primer (primer I) is essentially identical to primer binding section I, and the second primer (primer II) is essentially complementary to primer binding section II, wherein said first or said second primer is labelled, provided that:
- primer II is labelled if the ZipComcode is located on the first nucleic acid probe,
- primer I is labelled if the ZipComcode is located on the second nucleic acid probe, or
- primer I is labelled if the ZipComcode is located on the first nucleic acid probe,
(c4) amplifying any connected probe nucleic acid, wherein amplification is initiated by binding of a nucleic acid primer specific for a primer binding section,
thereby providing amplified target nucleic acids,
(d) hybridising the amplified target nucleic acids of step (c) to a capture probe, which is present on a microarray, and comprises a region essentially complementary to the ZipComcode (Zipcode), and,
(e) detecting the hybridised target nucleic acids of step (d), whereby the presence of micro-organisms is determined.

2. The method according to claim 1, wherein said first nucleic acid probe and/or said second nucleic acid probe hybridises to a genetic marker.

3. The method according to any of the claims 1 and 2, wherein 4 variants of said first nucleic acid probe are provided, said 4 variants being substantially identical except that each of the 4 variants containing a different nucleotide at its ultimate 3' end and each of the 4 variants containing a different primer binding site I.

4. The method according to any of the claims 1 to 3, wherein at least two groups of pairs of first and second nucleic acid probes are provided, wherein each group of first and second nucleic acid probes hybridises to a specific target nucleic acid, and comprises a specific primer binding site I and/or II.

5. The method according to any of the claims 1 to 4, wherein at least two groups of pairs of first and second nucleic acid probes are provided, wherein each group of first and second nucleic acid probes hybridises to a specific target nucleic acid, and the first nucleic acid probe of each group comprises a specific ZipComcode.

6. The method according to any of the claims 1 to 5, wherein said micro-organism is selected from the group consisting of eukaryotic, prokaryotic and/or viral micro-organisms.

7. The method according to any of claims 1 to 6, wherein said micro-organism is selected from the group consisting of food borne and waterborne micro-organisms.

8. The method according to any of the claims 1 to 7, wherein said micro-organism is selected from the group consisting of *Escherichia, Salmonella, Shigella, Mycobacterium, Lactobacillus, Lactococcus, Listeria*, *Leuconostoc, Bacillus, Staphylococcus, Clostridium*, *Vibrio, Enterococcus, Enterobacter, Yersinia, Legionella, Campylobacter*, *Streptococcus, Micrococcus, Pseudomonas*, *Flavobacterium*, *Alcaligenes, Microbacterium*, *Acinetobacter*, *Enterobacteriaceae*/*Coliforms*, *Aspergillus, Neurospora, Geotrichum, Blakeslea, Penicillium, Rhizomucor, Rhizopus*, *Trichoderma*, *Kluyveromyces*, *Candida*, *Hansenula*, *Rhodotorula*, *Torulopsis, Trichosporon* and *Saccharomyces*.

9. The method according to any of claims 1 to 8, wherein said step (a) is preceded by an enrichment of micro-organisms, comprising:
(a) growth of said micro-organisms on selective media, or
(b) growth of said micro-organisms on non-selective media.

10. The method according to any of claims 1 to 9, wherein said step (a) is preceded by an enrichment of micro-organisms, comprising concentrating the micro-organisms.

11. The method according to any of claims 1 to 10, wherein said step of capturing of micro-organisms is chosen from the group consisting of:
(a) filtering of an aqueous solution, whereby all particles larger than the sieving size are being captured,
(b) capturing of micro-organisms by antibodies,
(c) capturing of micro-organisms by ligands,
(d) centrifugation,
(e) sedimentation,
(f) electrostatic forces,
(g) coagulation, and
(h) flocculation.

12. The method according to claim 11, wherein said filtering comprises the use of a filter having a pore size of about between 0.15 and 1.4 µm, and preferably between about 0.5 and 1.2 µm.

13. The method according to any of claims 11 or 12, wherein said filtering comprises the use of a filter comprising a hollow fibre ceramic membrane or silicon nitride.

14. The method according to any of claims 11 to 13, wherein said filtering comprises the use of an Aquamarijn® filter or a CEPAration® filter.

15. The method according to any of the claims 1 to 14, wherein said capturing is followed by separating the micro-organisms from the remainder of the sample.

16. The method according to any of claims 1 to 15, wherein said extracting nucleic acids from said micro-organisms comprises lysing the micro-organisms.

17. The method according to claim 16, wherein said lysing is chosen from the group consisting of a treatment with a lysozyme, a pectinolytic, or guanidinium thiocyanate or by a mechanical treatment such as sonication or the use of a bead beater, by injecting the micro-organisms in hot phenol, and snap freezing the micro-organisms in liquid nitrogen followed by a mechanical treatment.

18. The method according to any of the claims 1 to 17, further comprising inactivating RNAses.

19. The method according to any of the claims 1 to 18, wherein said nucleic acids are chosen from the group consisting of DNA, rRNA, tRNA, mRNA, total RNA and tmRNA.

20. The method according to any of the claims 1 to 19, wherein said ZipComcode is located on the first nucleic acid probe in between the target-specific sequence I and the primer binding sequence I.

21. The method according to any of the claims 1 to 20, wherein said first nucleic acid probe is coupled with its 5' end to the 3' end of said second nucleic acid probe, possibly via a stuffer region.

22. The method according to any of the claims 1 to 21, wherein said connecting step (c3) comprises the use of a ligase.

23. The method according to any of claims 19 to 22, wherein said rRNA, tRNA, mRNA, total RNA, or tmRNA is converted to cDNA.

24. The method according to any of claims 1 to 23, wherein said nucleic acid or said cDNA is amplified using an amplification technique selected from the group consisting of PCR and LCR,.

25. The method according to any of the claims 1 to 24, wherein the amplified target nucleic acid is labelled.

26. The method according to claim 25, wherein the amplified target nucleic acid is labelled during amplification.

27. The method according to any of claims. 1 to 26, wherein said amplification is multiplex amplification.

28. The method according to any of claims 1 to 27, wherein said first primer is labelled at its 5' end.

29. The method according to any of the claims 1 to 28, wherein said label is a fluorescent or a phosphorescent label.

30. The method according to any of claims 1 to 29, wherein said fluorescent or phosphorescent label is chosen from the group consisting of FAM, TET, JOE, NED, HEX, (ET-)ROX, FITC, Cy2, Cy3, Cy5, Texas Red, TAMRA, Alexa, fluor 488^{™}, Biodipy^{™} FL, Rhodamine 123, R6G, Biodipy 530, Alexafluor^{™}532 and IRDyes^{™}.

31. The method according to any of the claims 1 to 30, wherein said capture probe hybridises specifically to said amplified target nucleic adds.

32. The method according to any of the claims 1 to 31, wherein said capture probe comprises a Zipcode which is essentially complementary to a corresponding ZipComcode.

33. The method according to any of the claims 1 to 32, wherein said Zipcode hybridises specifically to a corresponding ZipComcode.

34. The method according to any of the claims 1 to 33, wherein said capture probe is spatially addressable on said microarray.

35. The method according to any of the claims 1 to 34, wherein said microarray is a flow-through microarray.

36. The method according to any of the claims 1 to 35, wherein said microarray is a Pamchip®.

37. The method according to any of the claims 1 to 36, wherein a signal is detected after hybridising the specifically amplified nucleic acids to the capture probe.

38. The method according to claim 37, wherein said signal is a fluorescent or a phosphorescent signal, and said fluorescent or phosphorescent signal is detected by a CCD camera or by laser scanning, for example an FD10 system® or a Pamalyzer®.

39. The method according to any of claims 1 to 38, wherein the amplified target nucleic acids derived from at least two samples are hybridised to capture probes present on a single microarray.

40. The method according to any of claims 1 to 39, wherein the amplified target nucleic acids hybridised to the corresponding capture probes on a flow-through microarray results in a hybridisation pattern.

41. The method according to claim 40, wherein said hybridisation pattern is compared to hybridisation patterns stored in a databank.

42. A kit for determining the presence of micro-organisms in a sample, comprising a filter, means for capturing micro-organisms, means for extracting nucleic acids from said micro-organisms, means for specifically amplifying said nucleic acids, comprising the probes and primers as defined in claim 1, a microarray comprising capture probes with Zipcodes, means for analysing the amplified nucleic acids, and an instruction manual.

43. The kit according to claim 42, wherein said microarray is a flow-through microarray.

44. Use of at least one pair of a first nucleic acid probe and a second nucleic add probe as defined in any of the claims 1 to 5 in the method according to any of claims 1 to 41.

45. Use of a filter as defined in any of the claims 12 to 14 in the method according to any of claims 1 to 41.

46. Use of at least one set of two primers as defined in any of the claims 28 to 30 in the method according to any of claims 1 to 41.

## Patentansprüche

1. Verfahren zum Bestimmen der Anwesenheit von Mikroorganismen in einer Probe, umfassend die Schritte:
(a) Einfangen der Mikroorganismen, falls vorhanden,
(b) Extrahieren der Nukleinsäuren aus den Mikroorganismen, wobei die Nukleinsäuren Zielnukleinsäuren umfassen,
(c) Durchführen einer Ligasedetektionsreaktion (LDR) der Zielnukleinsäuren, umfassend:
(c1) Bereitstellen eines Paares einer ersten Nukleinsäure-Sonde und einer zweiten Nukleinsäure-Sonde, wobei die erste Nukleinsäure-Sonde eine zielspezifische Sequenz I in 3'-Position umfasst, die zu einem bestimmten Teil der Zielnukleinsäure komplementär ist, und wobei die zweite Nukleinsäure-Sonde eine zielspezifische Sequenz II in 5'-Position umfasst, die zu einem zweiten Teil der Zielnukleinsäure komplementär ist, die im Wesentlichen neben und 3' von der zielspezifischen Sequenz I positioniert ist, wobei die erste Nukleinsäure-Sonde weiterhin einen Primer-Bindungsabschnitt I (PBS(I)) in 5'-Position und gegebenenfalls einen Stuffer umfasst, und die zweite Nukleinsäure-Sonde einen Primer-Bindungsabschnitt II (PBS(II)) in 3'-Position und gegebenenfalls einen Stuffer umfasst; und wobei die erste Nukleinsäure-Sonde oder die zweite Nukleinsäure-Sonde weiterhin eine Region (ZipComcode) umfasst, welche (i) im Wesentlichen zu einer entsprechenden Region auf einer Fang-Sonde auf einem Mikroarray nach der Amplifizierung komplementär ist und (ii) im Wesentlichen zu der Zielnukleinsäure nicht komplementär ist, und (iii) welche zwischen der zielspezifischen Sequenz und dem Primer-Bindungsabschnitt positioniert ist;
(c2) Inkubieren der Zielnukleinsäure mit der ersten Nukleinsäure-Sonde und der zweiten Nukleinsäure-Sonde unter Bedingungen, die Hybridisierung der komplementären Nukleinsäuren ermöglichen,
(c3) Verbinden beliebiger, im Wesentlichen nebeneinander liegender Sonden,
(c4) Bereitstellen von mindestens einem Satz von zwei Primern, wobei der erste Primer (Primer I) im Wesentlichen zu dem Primer-Bindungsabschnitt I identisch ist, und der zweite Primer (Primer II) im Wesentlichen zu dem Primer-Bindungsabschnitt II komplementär ist, wobei der erste oder der zweite Primer markiert ist, unter der Voraussetzung, dass:
- Primer II markiert ist, wenn der ZipComcode auf der ersten Nukleinsäure-Sonde positioniert ist,
- Primer I markiert ist, wenn der ZipComcode auf der zweiten Nukleinsäure-Sonde positioniert ist, oder
- Primer I markiert ist, wenn der ZipComcode auf der ersten Nukleinsäure-Sonde positioniert ist,
(c4) Amplifizieren einer beliebigen gebundenen Nukleinsäure-Sonde, wobei Amplifizierung durch Binden eines Nukleinsäure-Primers gestartet wird, der für einen Primer-Bindungsabschnitt spezifisch ist, wodurch amplifizierte Zielnukleinsäuren bereitgestellt werden,
(d) Hybridisieren der amplifizierten Zielnukleinsäuren aus Schritt (c) mit einer Fang-Sonde, welche auf einem Mikroarray vorliegt, und eine Region umfasst, die im Wesentlichen zu dem ZipComcode (Zipcode) komplementär ist, und
(e) Nachweisen der hybridisierten Zielnukleinsäuren aus Schritt (d), wobei die Anwesenheit von Mikroorganismen bestimmt wird.

2. Verfahren nach Anspruch 1, wobei die erste Nukleinsäure-Sonde und/oder die zweite Nukleinsäure-Sonde mit einem genetischen Marker hybridisiert.

3. Verfahren nach einem der Ansprüche 1 und 2, wobei 4 Varianten der ersten Nukleinsäure-Sonde bereitgestellt werden, wobei die 4 Varianten im Wesentlichen identisch sind mit der Ausnahme, dass jede der 4 Varianten ein unterschiedliches Nukleotid an dessen äußerem 3'-Ende enthält und jede der 4 Varianten eine unterschiedliche Primer-Bindungsstelle I enthält.

4. Verfahren nach einem der Ansprüche 1 bis 3, wobei mindestens zwei Gruppen von Paaren der ersten und zweiten Nukleinsäure-Sonden bereitgestellt werden, wobei jede Gruppe der ersten und zweiten Nukleinsäure-Sonden mit einer spezifischen Zielnukleinsäure hybridisiert, und eine spezifische Primer-Bindungsstelle I und/oder II umfasst.

5. Verfahren nach einem der Ansprüche 1 bis 4, wobei mindestens zwei Gruppen von Paaren der ersten und zweiten Nukleinsäure-Sonden bereitgestellt werden, wobei jede Gruppe der ersten und zweiten Nukleinsäure-Sonden mit einer spezifischen Zielnukleinsäure hybridisiert, und die erste Nukleinsäure-Sonde jeder Gruppe einen spezifischen ZipComcode umfasst.

6. Verfahren nach einem der Ansprüche 1 bis 5, wobei der Mikroorganismus ausgewählt ist aus der Gruppe bestehend aus eukaryotischen, prokaryotischen und/oder viralen Mikroorganismen.

7. Verfahren nach einem der Ansprüche 1 bis 6, wobei der Mikroorganismus ausgewählt ist aus der Gruppe bestehend aus durch Lebensmittel übertragenen und durch Wasser übertragenen Mikroorganismen.

8. Verfahren nach einem der Ansprüche 1 bis 7, wobei der Mikroorganismus ausgewählt ist aus der Gruppe bestehend aus *Escherichia, Salmonella, Shigella, Mycobacterium, Lactobacillus, Lactococcus, Listeria, Leuconostoc, Bacillus, Staphylococcus, Clostridium, Vibrio, Enterococcus, Enterobacter, Yersinia, Legionella, Campylobacter*, *Streptococcus*, *Micrococcus, Pseudomonas, Flavobacterium, Alcaligenes, Microbacterium, Acinetobacter*, *Enterobacteriaceae*/ *Coliforme, Aspergillus, Neurospora, Geotrichum, Blakeslea*, *Penicillium, Rhizomucor, Rhizopus, Trichoderma, Kluyveromyces*, *Candida, Hansenula, Rhodotorula*, *Torulopsis, Trichosporon* und *Saccharomyces*.

9. Verfahren nach einem der Ansprüche 1 bis 8, wobei dem Schritt (a) eine Anreicherung von Mikroorganismen vorausgeht, umfassend:
(a) Wachstum der Mikroorganismen auf selektiven Nährsubstraten oder
(b) Wachstum der Mikroorganismen auf nicht-selektiven Nährsubstraten.

10. Verfahren nach einem der Ansprüche 1 bis 9, wobei dem Schritt (a) eine Anreicherung von Mikroorganismen vorausgeht, umfassend das Konzentrieren der Mikroorganismen.

11. Verfahren nach einem der Ansprüche 1 bis 10, wobei der Schritt des Einfangens der Mikroorganismen ausgewählt ist aus der Gruppe bestehend aus:
(a) Filtrieren einer wässrigen Lösung, wobei alle Partikel eingefangen werden, die größer als die Siebgröße sind,
(b) Einfangen der Mikroorganismen durch Antikörper,
(c) Einfangen der Mikroorganismen durch Liganden,
(d) Zentrifugation,
(e) Sedimentation,
(f) elektrostatische Kräfte,
(g) Gerinnung und
(h) Ausflockung

12. Verfahren nach Anspruch 11, wobei das Filtrieren die Verwendung eines Filters mit einer Porengröße von etwa zwischen 0,15 und 1,4 µm und bevorzugt zwischen etwa 0,5 und 1,2 µm umfasst.

13. Verfahren nach einem der Ansprüche 11 oder 12, wobei das Filtrieren die Verwendung eines Filters umfasst, der eine hohle Keramikfasermembran oder Siliciumnitrid umfasst.

14. Verfahren nach einem der Ansprüche 11 bis 13, wobei das Filtrieren die Verwendung eines Aquamarijn®-Filters oder eines CEPAration®-Filters umfasst.

15. Verfahren nach einem der Ansprüche 1 bis 14, wobei dem Einfangen das Abtrennen der Mikroorganismen von dem Rest der Probe vorausgeht.

16. Verfahren nach einem der Ansprüche 1 bis 15, wobei das Extrahieren der Nukleinsäuren aus den Mikroorganismen das Lysieren der Mikroorganismen umfasst.

17. Verfahren nach Anspruch 16, wobei das Lysieren ausgewählt ist aus der Gruppe bestehend aus einer Behandlung mit einem Lysozym, einem pektinolytischen Enzym oder Guanidiniumthiocyanat oder durch mechanische Behandlung, wie zum Beispiel Beschallung oder die Verwendung eines Bead-Beaters, durch Injizieren der Mikroorganismen in heißes Phenol, und Schock-Gefrieren der Mikroorganismen in flüssigem Stickstoff und anschließend durch mechanische Behandlung.

18. Verfahren nach einem der Ansprüche 1 bis 17, weiterhin umfassend das Inaktivieren von RNAsen.

19. Verfahren nach einem der Ansprüche 1 bis 18, wobei die Nukleinsäuren ausgewählt sind aus der Gruppe bestehend aus DNA, rRNA, tRNA, mRNA, Gesamt-RNA und tmRNA.

20. Verfahren nach einem der Ansprüche 1 bis 19, wobei der ZipComcode auf der ersten Nukleinsäure-Sonde zwischen der zielspezifischen Sequenz I und der Primer-Bindungssequenz I positioniert ist.

21. Verfahren nach einem der Ansprüche 1 bis 20, wobei die erste Nukleinsäure-Sonde mit ihrem 5'-Ende an das 3'-Ende der zweiten Nukleinsäure-Sonde, gegebenenfalls über eine Stuffer-Region, gekoppelt ist.

22. Verfahren nach einem der Ansprüche 1 bis 21, wobei der Bindungsschritt (c3) die Verwendung einer Ligase umfasst.

23. Verfahren nach einem der Ansprüche 19 bis 22, wobei die rRNA, tRNA, mRNA, Gesamt-RNA oder tmRNA zu cDNA umgewandelt wird.

24. Verfahren nach einem der Ansprüche 1 bis 23, wobei die Nukleinsäure oder die cDNA unter Verwendung einer Amplifizierungsmethode amplifiziert wird, die ausgewählt ist aus der Gruppe bestehend aus PCR und LCR.

25. Verfahren nach einem der Ansprüche 1 bis 24, wobei die amplifizierte Zielnukleinsäure markiert ist.

26. Verfahren nach Anspruch 25, wobei die amplifizierte Zielnukleinsäure während der Amplifizierung markiert wird.

27. Verfahren nach einem der Ansprüche 1 bis 26, wobei die Amplifizierung eine Multiplex-Amplifizierung ist.

28. Verfahren nach einem der Ansprüche 1 bis 27, wobei der erste Primer an seinem 5'-Ende markiert ist.

29. Verfahren nach einem der Ansprüche 1 bis 28, wobei die Markierung eine Fluoreszenz- oder eine Phosphoreszenz-Markierung ist.

30. Verfahren nach einem der Ansprüche 1 bis 29, wobei die Fluoreszenz- oder Phosphoreszenz-Markierung ausgewählt ist aus der Gruppe bestehend aus FAM, TET, JOE, NED, HEX, (ET-)ROX, FITC, Cy2, Cy3, Cy5, Texasrot, TAMRA, Alexa, Fluor 488®, Biodipy® FL, Rhodamin 123, R6G, Biodipy 530, Alexafluor® 532 und IRDyes®.

31. Verfahren nach einem der Ansprüche 1 bis 30, wobei die Fang-Sonde mit den amplifizierten Zielnukleinsäuren spezifisch hybridisiert.

32. Verfahren nach einem der Ansprüche 1 bis 31, wobei die Fang-Sonde einen Zipcode umfasst, der im Wesentlichen zu einem entsprechenden ZipComcode komplementär ist.

33. Verfahren nach einem der Ansprüche 1 bis 32, wobei der Zipcode spezifisch mit einem entsprechenden ZipComcode hybridisiert.

34. Verfahren nach einem der Ansprüche 1 bis 33, wobei die Fang-Sonde auf dem Mikroarray räumlich adressierbar ist.

35. Verfahren nach einem der Ansprüche 1 bis 34, wobei der Mikroarray ein Durchfluss-Mikroarray ist.

36. Verfahren nach einem der Ansprüche 1 bis 35, wobei der Mikroarray ein Pamchip® ist.

37. Verfahren nach einem der Ansprüche 1 bis 36, wobei nach dem Hybridisieren der spezifisch amplifizierten Nukleinsäuren mit der Fang-Sonde ein Signal nachgewiesen wird.

38. Verfahren nach Anspruch 37, wobei das Signal ein Fluoreszenz- oder ein Phosphoreszenz-Signal ist, und das Fluoreszenz- oder Phosphoreszenz-Signal mit einer CCD-Kamera oder durch Laser-Scanning, zum Beispiel durch ein FD10-System® oder einen Pamalyzer®, nachgewiesen wird.

39. Verfahren nach einem der Ansprüche 1 bis 38, wobei die amplifizierten Zielnukleinsäuren, die von mindestens zwei Proben stammen, mit Fang-Sonden, die auf einem einzelnen Mikroarray vorliegen, hybridisiert werden.

40. Verfahren nach einem der Ansprüche 1 bis 39, wobei die amplifizierten Zielnukleinsäuren, die mit den entsprechenden Fang-Sonden auf einem Durchfluss-Mikroarray hybridisiert sind, ein Hybridisierungsmuster ergeben.

41. Verfahren nach Anspruch 40, wobei das Hybridisierungsmuster mit den in einer Datenbank gelagerten Hybridisierungsmustern verglichen wird.

42. Kit zum Bestimmen der Anwesenheit von Mikroorganismen in einer Probe, umfassend einen Filter, Mittel zum Einfangen von Mikroorganismen, Mittel zum Extrahieren von Nukleinsäuren aus den Mikroorganismen, Mittel zum spezifischen Amplifizieren der Nukleinsäuren umfassend die Sonden und Primer, die gemäß Anspruch 1 definiert sind, einen Mikroarray umfassend Fang-Sonden mit Zipcodes, Mittel zum Analysieren der amplifizierten Nukleinsäuren und eine Bedienungsanleitung.

43. Kit nach Anspruch 42, wobei der Mikroarray ein Durchfluss-Mikroarray ist.

44. Verwendung von mindestens einem Paar einer ersten Nukleinsäure-Sonde und einer zweiten Nukleinsäure-Sonde, die gemäß einem der Ansprüche 1 bis 5 definiert sind, in dem Verfahren gemäß einem der Ansprüche 1 bis 41.

45. Verwendung eines Filters, der gemäß einem der Ansprüche 12 bis 14 definiert ist, in dem Verfahren gemäß einem der Ansprüche 1 bis 41.

46. Verwendung von mindestens einem Satz von zwei Primern, die gemäß einem der Ansprüche 28 bis 30 definiert sind, in dem Verfahren gemäß einem der Ansprüche 1 bis 41.

## Revendications

1. Une méthode pour déterminer la présence de micro-organismes dans un échantillon, comprenant les étapes :
(a) de capture desdits micro-organismes si présent,
(b) d'extraction des acides nucléiques desdits micro-organismes, les dits acides nucléiques comprenant des acides nucléiques cibles,
(c) d'effectuer une réaction de détection par ligase sur lesdits acides nucléiques cibles, comprenant :
(c1) la fourniture d'une paire d'un premier acide nucléique sonde et d'un second acide nucléique sonde, ledit premier acide nucléique sonde comprenant une séquence I localisée en 3', spécifique d'une cible et complémentaire à une partie distincte dudit acide nucléique cible et ledit second acide nucléique sonde comprenant une séquence II localisée en 5' spécifique d'une cible et complémentaire à une seconde partie dudit acide nucléique cible localisée essentiellement adjacente à et en 3' de ladite séquence I spécifique d'une cible, dans laquelle ledit premier acide nucléique sonde comprend en outre un site de fixation de l'amorce I (PBS (I)) localisé en 5' et éventuellement un remplisseur, et ledit second acide nucléique sonde comprend un site de fixation de l'amorce II (PBS (II)) localisé en 3' et éventuellement un remplisseur ; et dans laquelle le premier acide nucléique sonde ou le second acide nucléique sonde comprend en outre une région (ZipComcode) qui est (i) essentiellement complémentaire d'une région correspondante d'une sonde de capture sur un microréseau d'amplification et (ii) essentiellement non-complémentaire dudit nucléique acide cible, et (iii) qui est localisé entre la séquence spécifique d'une cible et le site de fixation de l'amorce ;
(c2) l'incubation dudit acide nucléique cible avec ledit premier acide nucléique sonde et ledit second acide nucléique sonde, dans des conditions permettant l'hybridation des acides nucléiques complémentaires,
(c3) la connexion de toutes sondes adjacentes complémentaires, et
(c4) la fourniture d'au moins un jeu de deux amorces, dans laquelle la première amorce (amorce I) est essentiellement identique au site de fixation d'amorce I, et la seconde amorce (amorce II) est essentiellement complémentaire au site de fixation d'amorce II, dans laquelle ladite première et seconde amorce est marquée, à condition que :
- l'amorce II est marquée si le ZipComcode est localisé sur le premier acide nucléique sonde,
- l'amorce I est marquée si le ZipComcode est localisé sur le second acide nucléique sonde,ou
- l'amorce I est marquée si le ZipComcode est localisé sur le premier acide nucléique sonde,
(c4) l'amplification de tout acide nucléique sonde connecté, dans laquelle l'amplification est initiée par la liaison d'une amorce d'acide nucléique spécifique à un site de fixation d'amorce,
de sorte à fournir des acides nucléiques cibles amplifiés,
(d) d'hybrider les acides nucléiques cibles amplifiées à l'étape (c) à une sonde de capture, qui est présent sur un microréseau, et comprend une région essentiellement complémentaire au ZipComcode (Zipcode), et,
(e) de détecter les acides nucléiques cibles hybridés de l'étape (d), par laquelle la présence d'un micro-organisme est déterminée.

2. La méthode selon la revendication 1, dans laquelle ledit premier acide nucléique sonde et/ou le second acide nucléique sonde s'hybride à un marqueur génétique.

3. La méthode selon l'une quelconque des revendications 1 et 2, dans laquelle 4 variantes dudit premier acide nucléique sonde sont fournies, lesdites 4 variantes étant substantiellement identiques excepté que chacune des 4 variantes contient un nucléotide différent à son extrémité 3' ultime et chacune des 4 variantes contient un site de fixation d'amorce I différent.

4. La méthode selon l'une quelconque des revendications 1 à 3, dans laquelle au moins deux groupes de paires de premier et second acides nucléiques sondes sont fournis, dans laquelle chaque groupe de premier et second acides nucléiques sondes s'hybride à un acide nucléique cible spécifique, et comprend un site spécifique de fixation d'amorce I et/ou II.

5. La méthode selon l'une quelconque des revendications 1 à 4, dans laquelle au moins deux groupes de paires de premier et second acides nucléiques sondes sont fournis, dans laquelle chaque groupe de premier et second acides nucléiques sondes s'hybride à un acide nucléique cible spécifique, et le premier acide nucléique sonde de chaque groupe comprend un ZipComcode spécifique.

6. La méthode selon l'une quelconque des revendications 1 à 5, dans laquelle ledit micro-organisme est choisi dans le groupe consistant de micro-organismes eucaryotiques, procaryotiques et/ou viraux.

7. La méthode selon l'une quelconque des revendications 1 à 6, dans laquelle ledit micro-organisme est choisi dans le groupe consistant de micro-organismes d'origine hydrique et alimentaire.

8. La méthode selon l'une quelconque des revendications 1 à 7, dans laquelle ledit micro-organisme est choisi dans le groupe consistant de *Escherichia, Salmonella, Shigella, Mycobacterium, Lactobacillus, Lactococcus, Listeria, Leuconostoc, Bacillus*, *Staphylococcus, Clostridium, Vibrio, Enterococcus, Enterobacter, Yersinia, Legionella, Campylobacter*, *Streptococcus, Micrococcus, Pseudomonas, Flavobacterium, Alcaligenes, Microbacterium, Acinetobacter, Enterobacteriaceae*/*Coliforms, Aspergillus, Neurospora, Geotrichum, Blakeslea, Penicillium, Rhizomucor, Rhizopus, Trichoderma, Kluyveromyces, Candida, Hansenula, Rhodotorula, Torulopsis, Trichosporon* et *Saccharomyces.*

9. La méthode selon l'une quelconque des revendications 1 à 8, dans laquelle ladite étape (a) est précédée par un enrichissement des micro-organismes comprenant :
(a) la croissance desdits micro-organisme sur milieux sélectifs, ou
(b) la croissance desdits micro-organisme sur milieux non-sélectifs.

10. La méthode selon l'une quelconque des revendications 1 à 9, dans laquelle ladite étape (a) est précédée par un enrichissement des micro-organismes comprenant la concentration des micro-organismes.

11. La méthode selon l'une quelconque des revendications 1 à 10, dans laquelle ladite étape de capture des micro-organismes est choisie dans le groupe consistant de :
(a) la filtration d'une solution aqueuse, par laquelle toutes les particules plus larges que la taille de tamisage sont capturées,
(b) la capture de micro-organismes par des anticorps,
(c) la capture de micro-organismes par des ligands,
(d) la centrifugation,
(e) la sédimentation,
(f) les forces électrostatiques,
(g) la coagulation, et
(h) la floculation.

12. La méthode selon la revendication 11, dans laquelle ladite filtration comprend l'utilisation d'un filtre ayant une dimension de pore entre environ 0,15 et 1,4 µm, et de préférence entre environ 0,5 et 1,2 µm.

13. La méthode selon l'une quelconque des revendications 11 ou 12, dans laquelle ladite filtration comprend l'utilisation d'un filtre comprenant une membrane en céramique à fibre creuse ou du nitrure de silicium.

14. La méthode selon l'une quelconque des revendications 11 à 13, dans laquelle ladite filtration comprend l'utilisation d'un filtre Aquamarijn® ou d'un filtre CEPAration®.

15. La méthode selon l'une quelconque des revendications 1 à 14, dans laquelle ladite capture est suivie par la séparation des micro-organismes du reste de l'échantillon.

16. La méthode selon l'une quelconque des revendications 1 à 15, dans laquelle ladite extraction des acides nucléiques des micro-organismes comprend la lyse des micro-organismes.

17. La méthode selon la revendication 16, dans laquelle ladite lyse est choisie dans le groupe consistant d'un traitement avec un lysozyme, un pectinolitique, ou du thiocyanate de guanidinium ou d'un traitement mécanique tel que la sonification ou l'utilisation d'un moulin à billes, l'injection des micro-organismes dans du méthanol chaud, et la congélation ultra rapide des micro-organismes dans de l'azote liquide suivit d'un traitement mécanique.

18. La méthode selon l'une quelconque des revendications 1 à 17, comprenant en outre l'inactivation des ribonucléases.

19. La méthode selon l'une quelconque des revendications 1 à 18, dans laquelle lesdits acides nucléiques sont choisis dans le groupe consistant d'ADN, d'ARNr, d'ARNt, d'ARNm, d'ARN total et d'ARNtm.

20. La méthode selon l'une quelconque des revendications 1 à 19, dans laquelle ledit ZipComcode est localisé sur le premier acide nucléique sonde entre la séquence I spécifique à la cible et le site de fixation d'amorce I.

21. La méthode selon l'une quelconque des revendications 1 à 20, dans laquelle ledit premier acide nucléique sonde est couplé par son extrémité 5' à l'extrémité 3' dudit second acide nucléique sonde, éventuellement par le biais d'une région de remplissage.

22. La méthode selon l'une quelconque des revendications 1 à 21, dans laquelle ladite étape de connexion (c3) comprend l'utilisation d'une ligase.

23. La méthode selon l'une quelconque des revendications 19 à 22, dans laquelle ledit ADN, ARNr, ARNt, ARNm, ARN total ou ARNtm est converti en ADNc.

24. La méthode selon l'une quelconque des revendications 1 à 23, dans laquelle ledit acide nucléique ou ledit ADNc est amplifié en utilisant une technique d'amplification choisie dans le groupe consistant de la PCR et la LCR.

25. La méthode selon l'une quelconque des revendications 1 à 24, dans laquelle l'acide nucléique cible amplifié est marqué.

26. La méthode selon la revendication 25, dans laquelle l'acide nucléique cible amplifié est marqué pendant l'amplification.

27. La méthode selon l'une quelconque des revendications 1 à 26, dans laquelle ladite amplification est une amplification multiplex.

28. La méthode selon l'une quelconque des revendications 1 à 27, dans laquelle ladite première amorce est marquée à son extrémité 5'.

29. La méthode selon l'une quelconque des revendications 1 à 28, dans laquelle ledit marqueur est un marqueur fluorescent ou phosphorescent.

30. La méthode selon l'une quelconque des revendications 1 à 29, dans laquelle ledit marqueur fluorescent ou phosphorescent est choisi dans le groupe consistant de FAM, TET, JOE, NED, HEX, (ET-)ROX, FITC, Cy2, Cy3, Cy5, Texas Red, TAMRA, Alexa, fluor 488^{™}, Biodipy^{™} FL, Rhodamine 123, R6G, Biodipy 530, Alexafluor^{™}532 et IRDyes^{™}.

31. La méthode selon l'une quelconque des revendications 1 à 30, dans laquelle ladite sonde de capture s'hybride spécifiquement aux dits acides nucléiques cibles amplifiés.

32. La méthode selon l'une quelconque des revendications 1 à 31, dans laquelle ladite sonde de capture comprend un Zipcode qui est essentiellement complémentaire à un ZipComcode correspondant.

33. La méthode selon l'une quelconque des revendications 1 à 32, dans laquelle ledit Zipcode s'hybride spécifiquement à un ZipComcode correspondant.

34. La méthode selon l'une quelconque des revendications 1 à 33, dans laquelle ladite sonde de capture est adressée d'une façon spatiale sur ledit microréseau.

35. La méthode selon l'une quelconque des revendications 1 à 34, dans laquelle ledit microréseau est à flux continu.

36. La méthode selon l'une quelconque des revendications 1 à 35, dans laquelle ledit microréseau est un Pamchip®.

37. La méthode selon l'une quelconque des revendications 1 à 36, dans laquelle un signal est détecté après l'hybridation des acides nucléiques spécifiquement amplifiés à la sonde de capture.

38. La méthode selon la revendication 37, dans laquelle ledit signal est un signal fluorescent ou phosphorescent, et ledit signal fluorescent ou phosphorescent est détecté par une caméra DTC ou par scannage au laser, par exemple un système FD10® ou un Pamalyzer®.

39. La méthode selon l'une quelconque des revendications 1 à 38, dans laquelle les acides nucléiques amplifiés dérivés d'au mois deux échantillons sont hybridés à des sondes de captures présentes sur un seul microréseau.

40. La méthode selon l'une quelconque des revendications 1 à 39, dans laquelle les acides nucléiques cibles amplifiés hybridés aux sondes de captures correspondantes sur un microréseau à flux continu résultent en un modèle d'hybridation.

41. La méthode selon la revendication 40, dans laquelle ledit modèle d'hybridation est comparé à des modèles d'hybridation stockés dans une base de données.

42. Une trousse pour déterminer la présence de micro-organismes dans un échantillon, comprenant un filtre, des moyens de capture des micro-organismes, des moyens d'extraction des acides nucléiques desdits micro-organismes, un moyen pour amplifier spécifiquement lesdits acides nucléiques, comprenant les sondes et les amorces telles que définies à la revendication 1, un microréseau comprenant des sondes de captures avec Zipcodes, des moyens pour l'analyse des acides nucléiques amplifiés, et un guide d'instructions.

43. La trousse selon la revendication 42, dans laquelle ledit microréseau est un microréseau à flux continu.

44. Utilisation d'au moins une paire d'un premier acide nucléique sonde et un second acide nucléique sonde telle que définie dans l'une quelconque des revendications 1 à 5 dans la méthode selon l'une quelconque des revendications 1 à 41.

45. Utilisation d'un filtre tel que défini dans l'une quelconque des revendications 12 à 14 dans la méthode selon l'une quelconque des revendications 1 à 41.

46. Utilisation d'au moins un jeu de deux amorces tel que défini dans l'une quelconque des revendications 28 à 30 dans la méthode selon l'une quelconque des revendications 1 à 41.


### SEQUENCE LISTING (ANNEX)

<110> CHECK-POINTS BV
<120> Fast method for detecting micro-organisms in food samples
<130> CHE-001-PCT
<140> PCT/EP2004/005951
   <141> 2004-06-02
<150> EP 03447138.3
   <151> 2003-06-02
<160> 22
<170> PatentIn version 3.3
<210> 1
   <211> 16
   <212> DNA
   <213> Artificial
<220>
   <223> primer
<400> 1
   gactgcgtac caattc 16
<210> 2
   <211> 16
   <212> DNA
   <213> Artificial
<220>
   <223> primer
<400> 2
   gatgagtcct gagtaa 16
<210> 3
   <211> 15
   <212> DNA
   <213> Artificial
<220>
   <223> Primer
<400> 3
   gactgcgtac actag 15
<210> 4
   <211> 16
   <212> DNA
   <213> Artificial
<220>
   <223> Primer
<400> 4
   gatgagtcct gagcga 16
<210> 5
   <211> 19
   <212> DNA
   <213> Artificial
<220>
   <223> Primer
<400> 5
   gtagactgcg taccaattc 19
<210> 6
   <211> 19
   <212> DNA
   <213> Artificial
<220>
   <223> Primer
<400> 6
   gacgatgagt cctgagtaa 19
<210> 7
   <211> 24
   <212> DNA
   <213> Artificial
<220>
   <223> --
<400> 7
   tacatatcac aacgtgcgtg gagg 24
<210> 8
   <211> 24
   <212> DNA
   <213> Artificial
<220>
   <223> --
<400> 8
   cctcatgtca acgaagaaca gaac 24
<210> 9
   <211> 24
   <212> DNA
   <213> Artificial
<220>
   <223> --
<400> 9
   ttatggtgat cagtcaacca ccag 24
<210> 10
   <211> 24
   <212> DNA
   <213> Artificial
<220>
   <223> --
<400> 10
   tccatgcgct tgctcttcat ctag 24
<210> 11
   <211> 24
   <212> DNA
   <213> Artificial
<220>
   <223> --
<400> 11
   gccttacata catctgtcgg ttgt 24
<210> 12
<400> 12
   000
<210> 13
   <211> 24
   <212> DNA
   <213> Artificial
<220>
   <223> --
<400> 13
   acacatacga ttctgcgaac ttca 24
<210> 14
   <211> 24
   <212> DNA
   <213> Artificial
<220>
   <223> --
<400> 14
   ttacaggatg tgctcaacag acgt 24
<210> 15
   <211> 24
   <212> DNA
   <213> Artificial
<220>
   <223> --
<400> 15
   gctcacaata attgcatgag ttgc 24
<210> 16
   <211> 24
   <212> DNA
   <213> Artificial
<220>
   <223> --
<400> 16
   tcacgcactg actgacagac tgct 24
<210> 17
   <211> 75
   <212> DNA
   <213> Staphylococcus aureus
<400> 17
   ccaaaccaac aagcttgctt gttggggttg taggacactc tatacggagt tacaaaggac 60
   gacattagac gaatc 75
<210> 18
   <211> 75
   <212> DNA
   <213> Staphylococcus epidermidis
<400> 18
   ccaaaccaac aagcttgctt gttggggttg taggacactc tatacggagt tacaaaagaa 60
   catgttagac gaatc 75
<210> 19
   <211> 34
   <212> DNA
   <213> Staphylococcus aureus
<400> 19
   tgcctcaatg tttcctgctg taatcttttt tttt 34
<210> 20
   <211> 34
   <212> DNA
   <213> Staphylococcus epidermidis
<400> 20
   tgcctcaatg ttttcttgta caatcttttt tttt 34
<210> 21
   <211> 34
   <212> DNA
   <213> Staphylococcus saprophyticus
<400> 21
   tgcctcaatg ttttcttgtc tgatcttttt tttt 34
<210> 22
   <211> 38
   <212> DNA
   <213> Staphylococcus sp.
<400> 22
   ggttgytcga acgaacaacc ccaacatcct ctcagata 38